# EUROPÄISCHE PATENTSCHRIFT

(11) **EP 3 679 010 B1**
(45) Veröffentlichungstag und Bekanntmachung des Hinweises auf die Patenterteilung: **27.10.2021**
(21) Anmeldenummer: 18779204.9
(22) Anmeldetag: 07.09.2018
(51) Int. Cl.: C07C 229/60, C07C 237/04, C07C 211/63, A61K 31/245, A61P 11/16, A61P 25/28, A61P 29/00, A61P 35/00, A61K 9/06, A61K 9/00, A61K 9/20, A61K 9/48, A61K 33/00, A61K 47/02, A61K 47/06

(54) **KOHLENSÄURE-ADDUKTE**
CARBONIC ACID ADDUCTS
PRODUITS D'ADDITION D'ACIDE CARBONIQUE

(30) Priorität: 07.09.2017 DE 102017120564
(43) Veröffentlichungstag der Anmeldung: 15.07.2020
(73) Patentinhaber: inflamed pharma GmbH, 07745 Jena (DE)
(72) Erfinder: ENGERT, Beatrice, 07745 Jena (DE); VOGELSANG, Susanne, 07745 Jena (DE)
(74) Vertreter: Schiweck Weinzierl Koch Patentanwälte Partnerschaft mbB
(86) Internationale Anmeldenummer: PCT/EP2018/074089
(87) Internationale Veröffentlichungsnummer: WO 2019/048590

(56) Entgegenhaltungen:
- WO-A1-2018/122626
- CN-A- 102 973 494
- DE-A1-102004 035 808
- DE-A1-102013 015 035
- GB-A- 349 640

## Beschreibung

### Technisches Gebiet

Der Gegenstand der vorliegenden Erfindung betrifft ein Kohlensäure Addukt (KA), umfassend Kohlensäure, mindestens ein Amin und optional mindestens ein Salz, herstellbar nach einem Verfahren umfassend die Schritte a) Bereitstellung einer Lösung (A), die gelöstes CO₂ umfasst, optional b) Auflösen einer Base (BA), die nicht dem Amin (AM) entspricht, in der Lösung (A) unter Erhalt der Lösung (A1), c) Lösen des mindestens einen Amins (AM) in der Lösung (A) oder (A1), unter Erhalt der Lösung (B), d) Einfrieren der nach Abschluss des Schrittes c) erhaltenen Lösung und e) Lagerung der in Schritt d) gefrorenen Lösung bei - 100 bis 0 °C für nicht länger als 4 Tage. Wobei der Gehalt an CO₂ in der Lösung, die dem Schritt c) unterzogen wird, mindestens 6 g/l beträgt. Weiterhin betrifft die Erfindung ein Verfahren zur Herstellung des Kohlensäure-Addukts (KA), eine pharmazeutische Zubereitung (PZ) umfassend das Kohlensäure-Addukt (KA), sowie Verfahren zu deren Herstellung und die Verwendung des Kohlensäure-Addukts (KA) oder der pharmazeutischen Zubereitung (PZ) in der Therapie für eine Reihe von Indikationen.

### Technischer Hintergrund

Wie in WO2006/007835 A2 offenbart wird, sind Kohlensäure-Addukte von Aminen, vor allem für pharmazeutisch-medizinische Anwendungen von Interesse. Insbesondere für das Kohlensäure-Addukt des Procains ist aus dieser Offenbarung bekannt, dass der Transport des Wirkstoffes, die Bioverfügbarkeit und die Verträglichkeit, unter anderem auch bei Acidosen verbessert wird. Ebenso wird eine im Vergleich zum Procain ausbleibende Hämolyse beobachtet. Ursache hierfür ist das Vorliegen eines Säure-Base Paares aus Amin und Kohlensäure, das eine hohe Pufferkapazität aufweist und gut löslich ist. Es wird außerdem erläutert, dass es von Bedeutung für die Stabilität des Procainiumhydrogencarbonats ist, die Konzentration von basischen Stoffen, wie Procain oder Carbonat etwa in Infusionslösungen desselben, niedrig zu halten. Die Basizität des Carbonates fördert die Verseifung des Procains. Außerdem katalysieren schon geringe Mengen an Carbonat den Zerfall des Procainiumhydrogencarbonats in Procain und Diprocainiumcarbonat. Als eine Möglichkeit zur Stabilisierung des Procainiumhydrogencarbonats oder Lidocainiumhydrogencarbonates wird der Einbau in Mineralsalze, wie Natriumchlorid und/ oder Dextrane, Stärke oder Cellulose zu Clathraten oder Clustern genannt. Weiterhin ist der Zusatz von CO₂ förderlich, wodurch der pH-Wert abgesenkt und der Anteil des Procainiumhydrogencarbonats nahezu auf 100 % gebracht wird. Durch den Einsatz des Procainiumhydrogencarbonats als Salzcluster in Form eines Pulvers, als Kapseln oder Tabletten wird die Verträglichkeit noch weiter erhöht. Eine Umhüllung der Tabletten wegen möglicher Zersetzung bei der Magen-Darm-Passage ist nicht unbedingt erforderlich, da sich beim Pressvorgang eine Schutzschicht bildet, die vorzugsweise im Darm aufgelöst wird. Als weitere Vorteile der Procainiumhydrogencarbonat-Salzcluster werden unter anderem deren nasale Anwendbarkeit und deren systemische oder lokale Anwendbarkeit als Antiinflammatorikum, als nebenwirkungsärmerer Ersatz für Corticoide beschrieben.

Ziel der Anmeldung DE 10 2013 015 035 A1, die direkt Bezug auf WO2006/007835 A2 nimmt, ist es, einige Nachteile der oben beschriebenen Procainium-Kohlensäure-Mineralsalzcluster durch Entwicklung geeigneter Formulierungen so zu verbessern, dass diese arzneimitteltauglich werden und damit höheren Ansprüchen bezüglich Stabilität, Wirkung und Akzeptanz genügen. Zu diesem Zweck wird ein Verfahren zur Herstellung von Kohlensäure-Mineralsalzclustern des Procains beschrieben, sowie dessen Verwendung in Formulierungen für Injektionslösungen, Inhalationslösungen, Salben und Tabletten. Analog zu WO2006/007835 A2 offenbart das Dokument, dass bei der Herstellung der Procainium-Kohlensäure-Mineralsalzcluster sowohl Salz als auch CO₂ in der Reaktionslösung anwesend sind.

Kohlensäure-Addukte, umfassend Kohlensäure, mindestens ein Amin und optional mindestens ein Salz, die besonders lange stabil sind und insbesondere eine hohe Lagerstabilität aufweisen, sind im Stand der Technik aber bisher nicht beschrieben worden. Auch der Einfluss einzelner Parameter auf die Lagerstabilität wird nicht diskutiert. Derartige Kohlensäure-Addukte wären für die kommerzielle Anwendung aber wünschenswert, da die beginnende Zersetzung der Addukte in ihre Ausgangsamine diese für die pharmazeutisch-medizinische Anwendung in vielen Fällen unbrauchbar macht. Ohne ausreichende Lagerstabilität müssen die entsprechenden Verbindungen vergleichsweise kurzfristig präpariert werden, was zusätzlich erschwert wird, wenn die Kohlensäure-Addukte für die Inverkehrbringung für medizinische-pharmazeutische Zwecke unter GMP-Bedingungen hergestellt werden müssen. Zusätzlich müssen sensiblere Verbindungen in eine andere Stabilitätskategorie (ICH (International Council for Harmonisation of Technical Requirements for Pharmaceuticals for Human Use)-Tabelle) eingestuft werden, das dann höhere Anforderungen an die Lager- und Transportbedingungen stellt und somit auch unter GMP (Good Manufacturing Practice)-Gesichtspunkten aufwendiger und kostenintensiver wird.

Demnach besteht das Ziel der vorliegenden Erfindung darin, ein weiteres Kohlensäure-Addukt bereitzustellen, das den höheren Anforderungen entspricht, sowie entsprechende pharmazeutische Zubereitungen zu entwickeln, die dieses Kohlensäure-Addukt umfassen.

### Zusammenfassung der Erfindung

Die Erfindung betrifft ein Kohlensäure-Addukt (KA) umfassend Kohlensäure, mindestens ein Amin (AM) und optional mindestens ein Salz (S),
herstellbar nach einem Verfahren umfassend die Schritte
a) Bereitstellung einer Lösung (A), die mindestens ein Lösungsmittel und in dem mindestens einem Lösungsmittel gelöstes CO₂ umfasst,
b) optional Auflösen einer Base (BA), die nicht dem Amin (AM) entspricht, in der Lösung (A) unter Erhalt der Lösung (A1),
c) Lösen des mindestens einen Amins (AM) in der Lösung (A) oder (A1), unter Erhalt der Lösung (B),
d) Einfrieren der nach Abschluss des Schrittes c) erhaltenen Lösung,
e) Lagerung der in Schritt d) eingefrorenen Lösung bei -100 bis 0 °C für nicht länger als 4 Tage

wobei der Gehalt an CO₂ in der Lösung, die dem Schritt c) unterzogen wird, mindestens 6 g/l, vorzugsweise mindestens 10 g/l, mehr bevorzugt mindestens 12 g/l, noch mehr bevorzugt mindestens 14 g/l und ganz besonders bevorzugt mindestens 15 g/l beträgt und das Amin (AM ) auch in Form eines Salzes eingesetzt werden kann.

Ein weiterer Aspekt der Erfindung stellt ein Verfahren zur Herstellung des Kohlensäure-Adduktes (KA), gemäß den vorstehend beschriebenen Schritten dar.

Außerdem umfasst die Erfindung ein Kohlensäure-Addukt (KA), umfassend mindestens ein Amin (AM), Kohlensäure und optional ein Salz (S), herstellbar gemäß dem vorstehend genannten Verfahren, wobei
i) das Kohlensäure-Addukt (KA), Procain, Kohlensäure und ein Salz (S) umfasst und der Zersetzungspunkt 65 bis 95 °C, vorzugsweise 70 bis 90 °C, mehr bevorzugt 85 bis 90 °C beträgt oder
ii) Lidocain, Kohlensäure und ein Salz (S) umfasst und der Zersetzungspunkt 20 bis 45 °C, vorzugsweise 25 bis 45 °C, mehr bevorzugt 30 bis 45 °C beträgt und /oder
iii) das Kohlensäure-Addukt (KA) bei Lagerung bei einer Temperatur von 2 bis 10 °C für mindestens 12 Monate, vorzugsweise für mindestens 13 Monate, mehr bevorzugt für mindestens 20 Monate, noch mehr bevorzugt für mindestens 23 Monate, besonders bevorzugt für mindestens 27 Monate stabil bleibt.

Weiterhin beinhaltet die Erfindung auch eine pharmazeutische Zubereitung (PZ) umfassend das Kohlensäure-Addukt (KA) gemäß einer der vorstehenden Definitionen, wobei
a) bei der Zubereitung der pharmazeutischen Zubereitung (PZ) optional die Temperatur weniger als 60 °C, vorzugsweise weniger als 50 °C, mehr bevorzugt 0 bis 50 °C beträgt und/oder
b) bei Anwendung von Dispergierern und/oder Salbenbereitern eine Drehzahl von < 2000 rpm verwendet wird sowie Verfahren und Kits zu deren Herstellung.

Darüber hinaus umfasst die Erfindung das Kohlensäure-Addukt (KA) oder die pharmazeutische Zubereitung zur Verwendung in einem Verfahren zur Anästhesie, zur Analgesie, zur begleitenden Behandlung von Krebs, zur Entzündungshemmung, zur Unterstützung der Wundheilung, insbesondere bei Verbrennungen, offenen Wunden und Narben, zur Behandlung von neurogenen Entzündungen wie Multiple Sklerose, MMN (Multifokale motorische Neuropathie), zur Behandlung von Sinusitis, zur Behandlung von Asthma, zur Behandlung von Rheumatischer Arthritis, zur Behandlung von Alzheimer, zur Behandlung von Demenz, zur Unterstützung der Rekonvaleszenz und zur Unterstützung des Anti-aging, zur Behandlung des Burn-out-Syndroms, zur Behandlung von Arthrose, zur Behandlung von Polyarthritis, zur Behandlung von Schmerzsyndrom und allgemeinen Schmerzen, zur prä- und postoperativen Behandlung (auch bei Knochenbrüchen), zur Präventiv- und Rehabilitationsmedizin, zur Behandlung von Zosterneuralgie, zur begleitenden Behandlung von Zosterneuralgie, zur Behandlung von Erkrankungen der Bauchorgane, wie Leber, Galle, Bauchspeicheldrüse, Darm, zur Behandlung von Magen-Darm-Erkrankungen (Collitis Ulcerosa, Morbus Krohn), zur Behandlung von Morbus Bechterew, zur Behandlung von chron. Schmerzen des Bewegungsapparats, zur Behandlung von Diabetes (Verbesserung der Blutzuckerwerte), zur Reduktion von Ödemen, zur Comedikation zu Opioden oder anderen Analgetika.

Das erfindungsgemäße Kohlensäure-Addukt (KA) zeichnet sich durch eine hohe Lagerstabilität und höhere Zersetzungstemperaturen aus. Ebenso weisen die Kohlensäure-Addukte (KA) eine signifikant höhere Lösungsgeschwindigkeit, insbesondere in Wasser auf, als die zugrundeliegenden Amine oder deren Salze.

Weiterhin zeigt das erfindungsgemäße Kohlensäure-Addukt (KA) ambiphile Eigenschaften, was es, beim Einsatz als Wirkstoff, besonders für Applikationsformen, vorzugsweise auch in Form einer pharmazeutischen Zubereitung (PZ), geeignet macht, die eine erhöhte Membrangängigkeit erfordern. Darüber hinaus erleichtert die Ambiphilie des Kohlensäure-Adduktes nach Eintritt in den Organismus auch den Transport desselben zum Wirkort, und erlaubt so Wirkstoff einzusparen.

Die Pufferwirkung, die durch die Anwesenheit des mindestens einen Amins (AM) und Kohlensäure erzielt wird, führt, bei Applikation des Kohlensäure-Addukts (KA) als Wirkstoff, zu einer geringeren Toxizität und besseren Verträglichkeit, als wenn das Salz des Amin alleine verabreicht wird.

### Kurze Beschreibung der Figuren

**Figur 1****:** Vergleichende DSC-Untersuchung der Beispiele 2.8 (Probe1), 2.7 (Probe 2) und 2.6 (Probe 3).
**Figur 2****:** Stabilitätsuntersuchung Hartgelatine Kapseln. Hartgelantine Kapseln mit 60 und 100 mg Wirkstoff, gemäß Tabelle 3, wie in Beispiel 4.1 im Detail beschrieben, wurden einer Stabilitätsuntersuchung über einen Zeitraum von 12 Monaten unterzogen.
**Figur 3****:** Membrangängigkeit des Kohlensäure-Adduktes (KA), umfassend Procain als Amin (AM) (KA-Procain) im Vergleich zu Procainhydrochlorid (ProcHCl). Wie in Beispiel 4.3 beschrieben, sind in Figur 3 die Konzentration der jeweils getesteten Substanz in einer Lösung nach Durchtritt durch einen Schweinedarm über die Zeit abgetragen.
**Figur 4****:** Vergleich der Stabilität einer 0,2%-igen Infusionslösung mit der Stabilität einer 2%-igen Injektionslösung. Wie in Beispiel 4.4.1 im Detail beschrieben ist, wurde die Stabilität von zwei parenteralen Lösungen über einen Zeitraum von 12 Monaten miteinander verglichen.
**Figur 5****:** Der Einfluss von CO₂ und Natriumchlorid auf die Stabilität einer 2%-igen Injektionslösung wurde bei Raumtemperatur über einen Zeitraum von 96 h verglichen. Nähere Details sind in Beispiel 4.4.2 beschrieben.

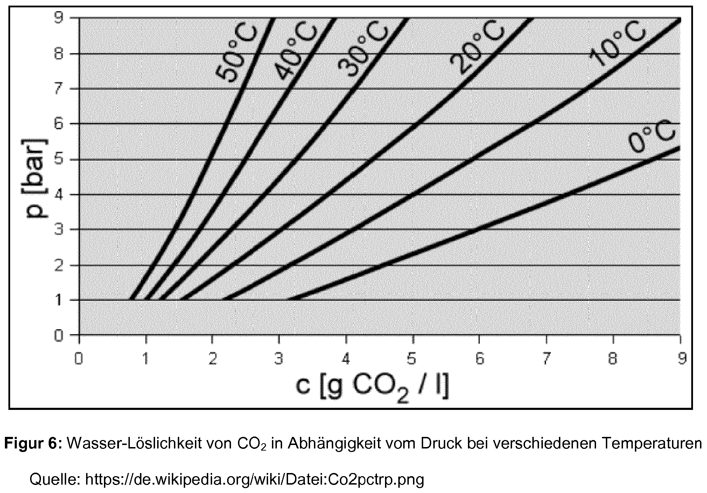

**Figur 6****:** Wasser-Löslichkeit von CO₂ in Abhängigkeit vom Druck bei verschiedenen Temperaturen.

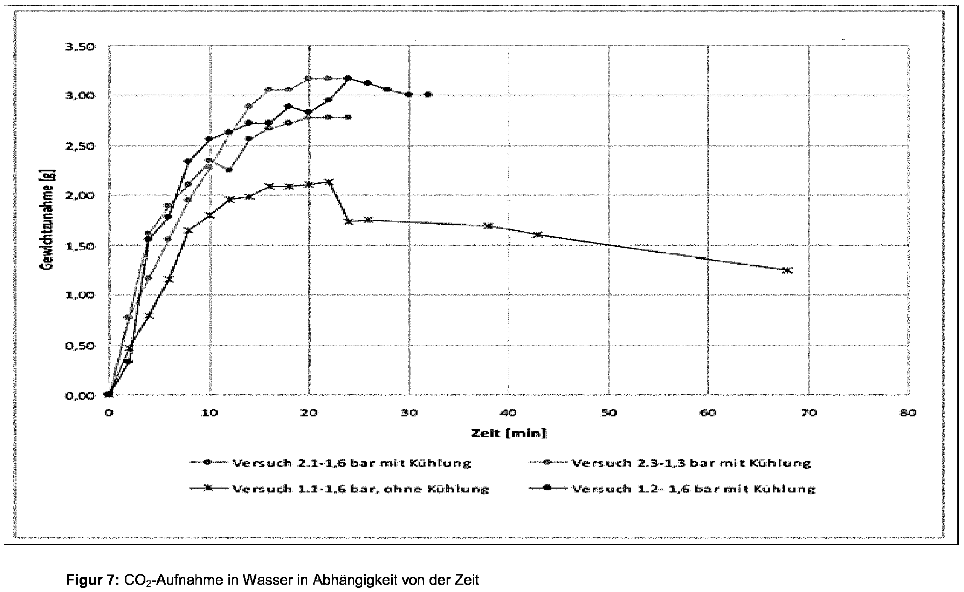

**Figur 7****:** CO₂-Aufnahme von Wasser in Abhängigkeit von der Zeit, in den Versuchen mit Kühlung betrug die Temperatur 8 °C.

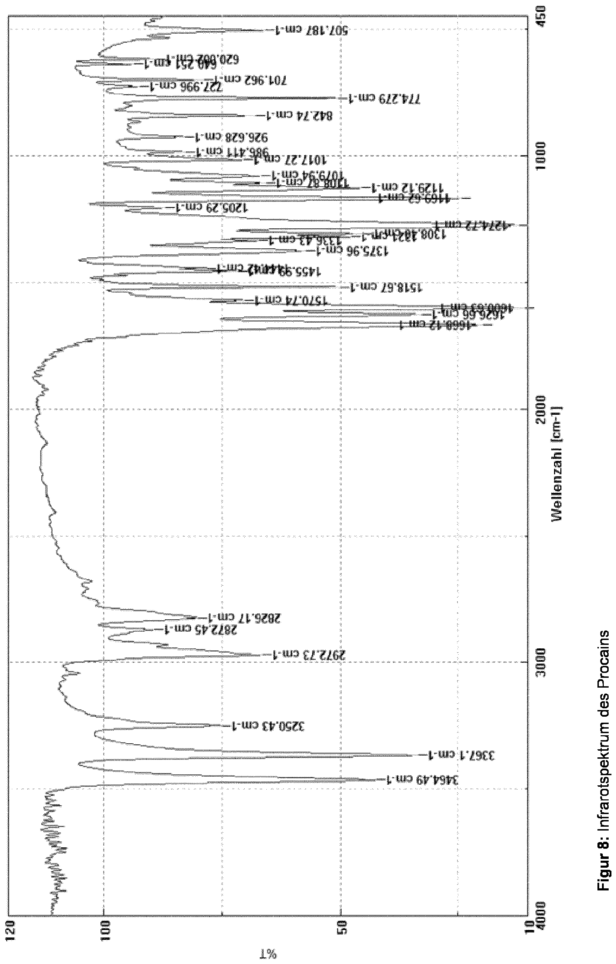

**Figur 8****:** Infrarotspektrum des Procains. Charakteristisch ist hier die Bande bei 3464 cm⁻¹.

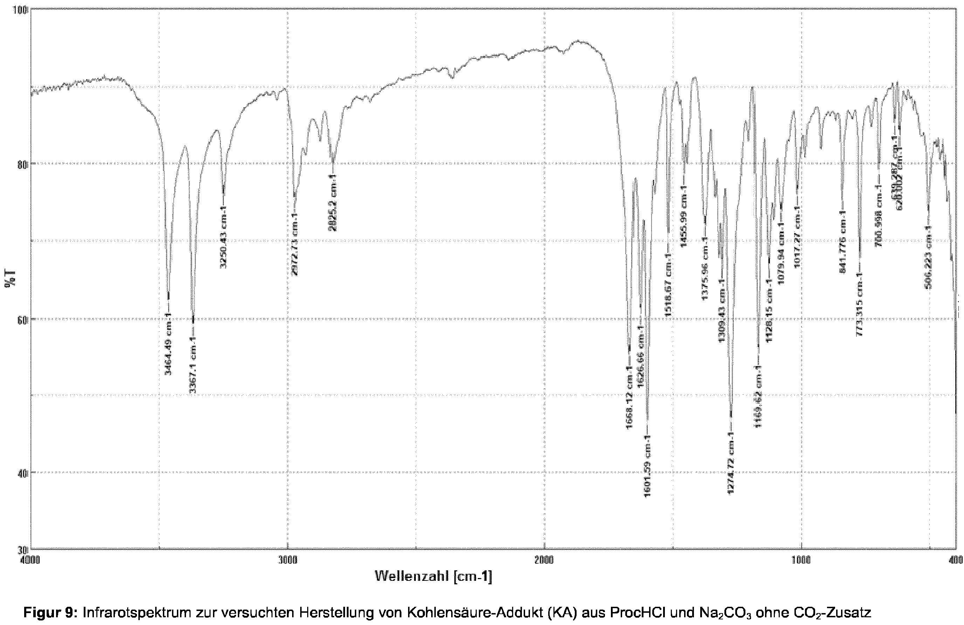

**Figur 9****:** Infrarotspektrum zur versuchten Herstellung von Kohlensäure-Addukt (KA) aus ProcHCl und Na₂CO₃ ohne CO₂-Zusatz. Die charakteristische Bande des Procains bei 3464 cm⁻¹ ist klar zu erkennen, während kein Kohlensäure-Addukt gebildet wird.

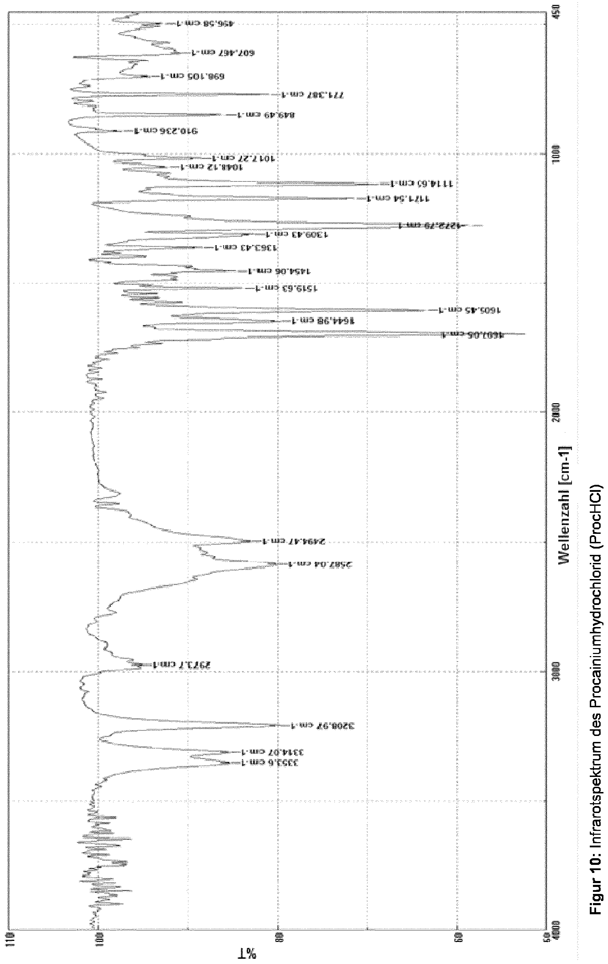

**Figur 10****:** Infrarotspektrum des Procainiumhydrochlorids.

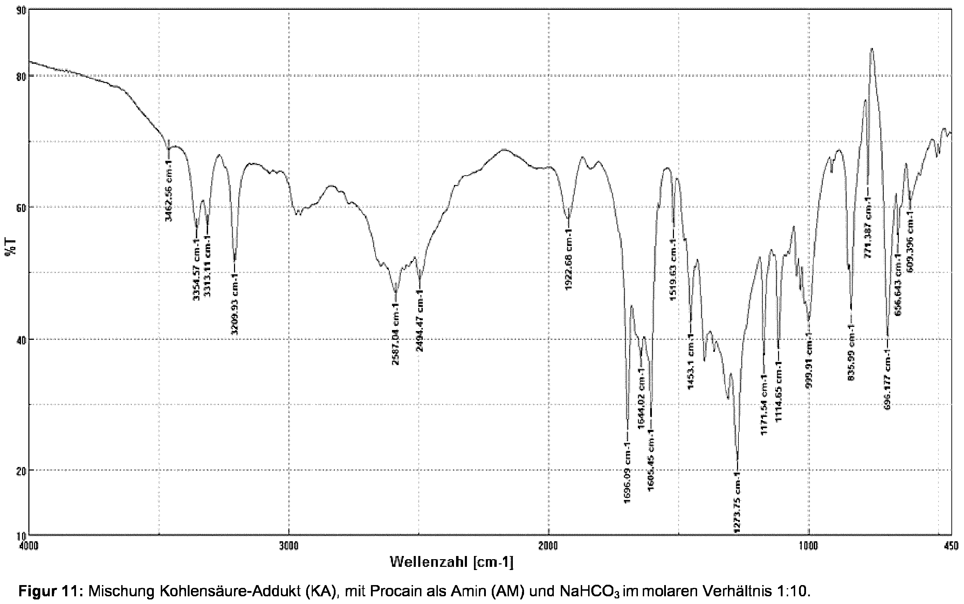

**Figur 11****:** Infrarotspektrum einer Feststoffmischung aus Kohlensäure-Addukt (KA), mit Procain als Amin (AM) und NaHCO₃ im molaren Verhältnis 1:10. Erkennbar ist die beginnende Procainbildung an der Bande 3462.56 cm⁻¹.

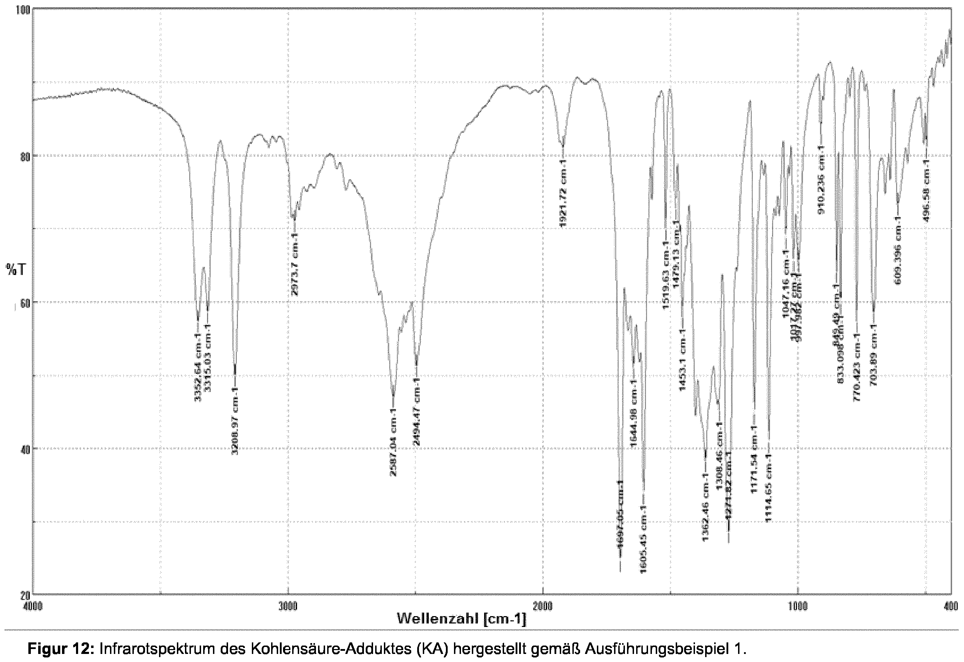

**Figur 12****:** Infrarotspektrum des Kohlensäure-Adduktes (KA) hergestellt gemäß Ausführungsbeispiel 1. Es ist keine Procainbande im Bereich 3461-3467 cm⁻¹ vorhanden.

### Detaillierte Beschreibung der Erfindung

Das Kohlensäure-Addukt (KA) umfasst Kohlensäure, mindestens ein Amin (AM) und optional mindestens ein Salz (S).

Unter einem Addukt wird im Rahmen dieser Erfindung ein Molekül verstanden, das durch Zusammenschluss von, bezogen auf ihr Molekulargewicht, kleineren Molekülen als das Addukt, über die Ausbildung von kovalenten oder nicht kovalenten Bindungen, vorzugsweise nicht kovalenten Bindungen gebildet wird. Vorzugsweise umfasst das Addukt als nicht kovalente Bindungen auch ionische Bindungen zwischen zumindest einem Teil der Teilchen, die sich zu dem Addukt zusammengeschlossen haben.

Kohlensäure H₂CO₃, kann in dem Kohlensäure-Addukt (KA) sowohl in nicht ionisierter Form, in teilionisierter Form oder auch vollständig ionisiert vorliegen. Diese unterschiedlichen lonisierungsgrade der Kohlensäure können in dem Kohlensäure-Addukt auch nebeneinander vorliegen. Die Ionisierung der Kohlensäure kann durch Übertragung eines oder beider Protonen der Kohlensäure auf ein anderes Molekül, vorzugsweise von mindestens einem Proton auf das Amin (AM) erfolgen.

Unter dem mindestens einem Amin (AM) wird ein Molekül verstanden, dass mindestens eine Aminogruppe, vorzugsweise eine Aminogruppe aufweist. Die Aminogruppe kann eine primäre, sekundäre oder tertiäre Aminogruppe sein.

Die mindestens eine Aminogruppe des Amins (AM) kann in dem Kohlensäure-Addukt (KA) sowohl in neutraler Form als auch in protonierter Form vorliegen. Wenn das Amin (AM) über mehr als eine Aminogruppe verfügt, können alle Aminogruppen in neutraler Form oder alle Aminogruppen in protonierter Form oder ein Teil der Aminogruppen in neutraler Form und ein Teil der Aminogruppen in protonierter Form vorliegen. Vorzugsweise liegt mindestens eine Aminogruppe in protonierter Form vor.

Vorzugsweise umfasst das Kohlensäure-Addukt mindestens ein Amin (AM) gemäß einer der nachstehenden Formeln (I) und/oder (II).

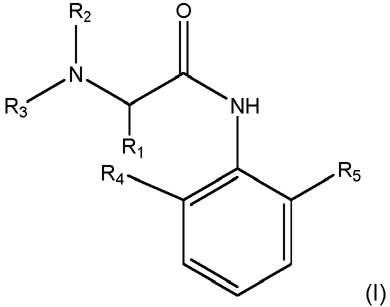

Wobei in Formel (I)
R₁ ist H, C₁₋₁₀ Alkyl, C₂₋₁₀ Alkenyl, Aryl oder Heteroaryl, vorzugsweise H oder C₁₋₁₀ Alkyl, mehr bevorzugt H oder C₁₋₄Alkyl;
R₂ ist H, C₁₋₁₀ Alkyl, C₂₋₁₀ Alkenyl, Aryl oder Heteroaryl, vorzugsweise H oder C₁₋₁₀ Alkyl, mehr bevorzugt H oder C₁₋₄Alkyl;
R₃ ist H, C₁₋₁₀ Alkyl, C₂₋₁₀ Alkenyl, Aryl oder Heteroaryl, vorzugsweise H oder C₁₋₁₀ Alkyl, mehr bevorzugt H oder C₁₋₄Alkyl;
wobei optional R₁ und R₃ miteinander verbunden sein können und zusammen mit dem Stickstoffatom an das R₃ gebunden ist und dem Kohlenstoffatom an das R₁ gebunden ist, einen gesättigten oder ungesättigten, vorzugsweise einen gesättigten Ring bilden können. Vorzugsweise ist der Ring 4, 5 oder 6 gliedrig, mehr bevorzugt 5 oder 6 gliedrig;
R₄ ist H, Halogen, C₁₋₁₀ Alkyl, C₂₋₁₀ Alkenyl, Aryl, Heteroaryl, vorzugsweise H oder Methyl;
R₅ ist H, Halogen, C₁₋₁₀ Alkyl, C₂₋₁₀ Alkenyl, Aryl oder Heteroaryl, vorzugsweise H, Methyl oder Halogen; 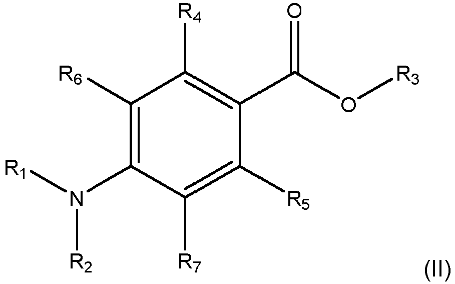
wobei in Formel (II)
   R₁ ist H, C₁₋₁₀ Alkyl, C₂₋₁₀ Alkenyl, Aryl oder Heteroaryl, vorzugsweise H oder C₁₋₁₀ Alkyl, mehr bevorzugt H;
   R₂ ist H, C₁₋₁₀ Alkyl, C₂₋₁₀ Alkenyl, Aryl oder Heteroaryl, vorzugsweise H oder C₁₋₁₀ Alkyl, mehr bevorzugt H;
   R₃ ist H, C₁₋₁₀ Alkyl, C₂₋₁₀ Alkenyl, Aryl, Heteroaryl oder -(CH₂)ₙNR₈R₉, vorzugsweise - (CH₂)ₙNR₈R₉;
   n ist 1 bis 5, vorzugsweise 1 bis 3, mehr bevorzugt 1 bis 2;
   R₈ ist C₁₋₁₀Alkyl, vorzugsweise C₁₋₂ Alkyl;
   R₉ ist C₁₋₁₀Alkyl, vorzugsweise C₁₋₂Alkyl;
   R₄ ist H, Halogen, C₁₋₁₀ Alkyl, C₂₋₁₀ Alkenyl, Aryl, Heteroaryl oder -O-C₁₋₁₀ Alkyl, vorzugsweise H, Halogen, C₁₋₁₀Alkyl oder -O-C₁₋₁₀Alkyl, mehr bevorzugt H oder Halogen;
   R₅ ist H, Halogen, C₁₋₁₀ Alkyl, C₂₋₁₀ Alkenyl, Aryl, Heteroaryl oder -O-C₁₋₁₀ Alkyl, vorzugsweise H, Halogen, C₁₋₁₀Alkyl oder -O-C₁₋₁₀Alkyl, mehr bevorzugt H oder Halogen;
   R₆ ist H, Halogen, C₁₋₁₀ Alkyl, C₂₋₁₀ Alkenyl, Aryl, Heteroaryl oder -O-C₁₋₁₀ Alkyl, vorzugsweise H, Halogen, C₁₋₁₀ Alkyl oder -O-C₁₋₁₀ Alkyl, mehr bevorzugt H oder -O-C₁₋₁₀ Alkyl;
   R₇ ist H, Halogen, C₁₋₁₀ Alkyl, C₂₋₁₀ Alkenyl, Aryl, Heteroaryl oder -O-C₁₋₁₀ Alkyl, vorzugsweise H, Halogen, C₁₋₁₀ Alkyl oder -O-C₁₋₁₀ Alkyl, mehr bevorzugt H oder -O-C₁₋₁₀ Alkyl.

In der vorliegenden Erfindung bedeuten Definitionen wie C₁₋₁₀ Alkyl, wie beispielsweise für den Rest R₁ von Formel (I) definiert, das dieser Substituent (Rest) ein gesättigter Alkylrest mit einer Kohlenstoffanzahl von 1 bis 10 ist. Der Alkylrest kann sowohl linear als auch verzweigt sowie gegebenenfalls zyklisch sein. Alkylreste, die sowohl eine zyklische als auch eine lineare Komponente aufweisen, fallen ebenfalls unter diese Definition. Gleiches gilt für andere Alkylreste wie beispielsweise ein C₁₋₂ Alkylrest. Gegebenenfalls können Alkylreste auch mit funktionellen Gruppen wie Amino, Hydroxy, Halogen, Aryl oder Heteroaryl einfach oder mehrfach substituiert sein. Soweit nicht anders angegeben, weisen Alkylreste vorzugsweise keine funktionellen Gruppen als Substituenten auf. Beispiele für Alkylreste sind Methyl, Ethyl, n-Propyl, n-Butyl, n-Pentyl, n-Hexyl, n-Heptyl, n-Octyl, n-Nonyl, iso-Propyl ( auch 2-Propyl oder 1-Methylethyl genannt), iso-Butyl, tert-Butyl, sec-Pentyl, neo-Pentyl, 1,2-Dimethylpropyl, iso-Amyl, iso-Hexyl, iso-Heptyl.

In der vorliegenden Erfindung bedeuten Definitionen wie C₂₋₁₀ Alkenyl, wie beispielsweise für den Rest R₁ von Formel (I) definiert, das dieser Substituent (Rest) ein Alkenylrest mit einer Kohlenstoffanzahl von 2 bis 10 ist, der mindestens eine ungesättigte Kohlenstoff-Kohlenstoffbindung aufweist. Der Alkenylrest kann sowohl linear als auch verzweigt sowie gegebenenfalls zyklisch sein. Alkenylreste, die sowohl eine zyklische als auch eine lineare Komponente aufweisen, fallen ebenfalls unter diese Definition. Gleiches gilt für andere Alkenylreste wie beispielsweise ein C₂₋₄ Alkenylrest. Gegebenenfalls können Alkenylreste auch mit funktionellen Gruppen wie Amino, Hydroxy, Halogen, Aryl-oder Heteroaryl einfach oder mehrfach substituiert sein. Soweit nicht anders angegeben, weisen Alkenylreste vorzugsweise keine funktionellen Gruppen als Substituenten auf. Beispiele für Alkenylreste sind Vinyl, 1-Propenyl, 2-Propenyl, 1-Butenyl, 2-Butenyl, 3-Butenyl, 1-Pentenyl, 2-Pentenyl, 3-Pentenyl, 4-Pentenyl, 1-Hexenyl, 2-Hexenyl, 3-Hexenyl, 4-Hexenyl, 5-Hexenyl, 1-Heptenyl, 3-Heptenyl, 4-Heptenyl, 1-Octenyl, 3-Octenyl, 5-Octenyl, 1-Nonenyl, 2,-Nonenyl.

In der vorliegenden Erfindung bedeutet Definitionen wie C₂₋₁₀ Alkenyl, wie beispielsweise für den Rest R₁ von Formel (I) definiert, das dieser Substituent (Rest) ein Alkenylrest mit einer Kohlenstoffanzahl von 2 bis 10 ist, der mindestens eine ungesättigte Kohlenstoff-Kohlenstoffbindung aufweist. Der Alkenylrest kann sowohl linear als auch verzweigt sowie gegebenenfalls zyklisch sein. Alkenylreste, die sowohl eine zyklische als auch eine lineare Komponente aufweisen, fallen ebenfalls unter diese Definition. Gleiches gilt für andere Alkenylreste wie beispielsweise ein C₂₋₄ Alkenylrest. Gegebenenfalls können Alkenylreste auch mit funktionellen Gruppen wie Amino, Hydroxy, Halogen, Aryl-oder Heteroaryl einfach oder mehrfach substituiert sein. Soweit nicht anders angegeben, weisen Alkenylreste vorzugsweise keine funktionellen Gruppen als Substituenten auf. Beispiele für Alkenylreste sind Vinyl, 1-Propenyl, 2-Propenyl, 1-Butenyl, 2-Butenyl, 3-Butenyl, 1-Pentenyl, 2-Pentenyl, 3-Pentenyl, 4-Pentenyl, 1-Hexenyl, 2-Hexenyl, 3-Hexenyl, 4-Hexenyl, 5-Hexenyl, 1-Heptenyl, 3-Heptenyl, 4-Heptenyl, 1-Octenyl, 3-Octenyl, 5-Octenyl, 1-Nonenyl, 2,-Nonenyl.

In der vorliegenden Erfindung bedeutet die Definition Aryl, dass es sich um einen aromatischen oder heteroaromatischen Rest handelt. Ein aromatischer Rest ist ein aromatischer cyclischer Kohlenwasserstoff, der aus einem Ring oder einem Ringsystem aus mehreren kondensierten Ringen bestehen kann. Der aromatische Rest kann beispielsweise monocyclisch, bicyclisch oder tricyclisch sein. Vorzugsweise bildet der monocyclische aromatische Rest einen 5- oder 6-gliedrigen Ring. Vorzugsweise bildet der bicyclische aromatische Ring einen 9- oder 10-gliedrigen Ring. Vorzugsweise bildet der tricyclische aromatische Ring einen 13- oder 14-gliedrigen Ring. Die Arylgruppe enthält vorzugsweise 3 bis 14, mehr bevorzugt 4 bis 6 Kohlenstoffatome. Gegebenenfalls können Arylreste auch mit funktionellen Gruppen wie Alkyl, Alkenyl, Amino, Hydroxy, Halogen, Aryl oder Heteroaryl einfach oder mehrfach substituiert sein. Beispiele für aromatische Reste sind Phenyl, und Naphtyl.

In der vorliegenden Erfindung bedeutet die Definition Heteroaryl, dass es sich um einen heteroaromatischen Rest handelt. Heteroaromatischer Ring bedeutet, dass in einem aromatischen Rest, wie vorstehend definiert, dessen Ringsystem durch Kohlenstoffatome gebildet wird, ein oder mehrere dieser Kohlenstoffatome durch Heteroatome wie O, N oder S ersetzt sind. Beispiele für heteroaromatische Reste, die in der vorliegenden Erfindung unter die Definition Aryl fallen, sind Furanyl, Thienyl, Oxazolyl, Pyrazolyl, Pyridyl und Indolyl.

In der vorliegenden Erfindung bedeutet die Definition Halogen, wie beispielsweise vorstehend für den Rest R₄ für Formel (I) definiert, dass es sich um einen Chlor-, Brom-, lod- oder Fluorsubstituenten handelt. Vorzugsweise handelt es sich um einen Chlor- oder Fluorsubstituenten.

Mehr bevorzugt umfasst das Kohlensäure-Addukt (KA) mindestens ein Amin (AM) ausgewählt aus der Gruppe 4-Aminobenzoesäure-2-(*N*,*N*-diethylamino-)ethylester (Procain), 4-Aminobenzoesäureethylester (Benzocain), 2-(Diethylamino)ethyl-4-amino-2-chlorbenzoat (Chlorprocain), 4-Amino-3-butoxybenzoesäure-2-diethylaminoethylester (Oxybuprocain), (2-(Dimethylamino)ethyl)-4-(butylamino)benzoat (Tetracain), *N*-[3-(4-Phenoxymethylphenyl)-propyl]morpholin (Fomocain), 2-Diethylamino-*N*-(2,6-dimethylphenyl)acetamid (Lidocain), (*RS*)-*N*-(2,6-Dimethylphenyl)-1-methylpiperidin-2-carboxamid (Mepivacain), *(RS)-N-(2-*Methylphenyl)-2-(propylamino)-propanamid (Prilocain), (*RS*)-4-Methyl-3-[2-(propylamino)-propanamido]thiophen-2-carbonsäuremethylester (Articain), (±)-1-Butyl-*N*-(2,6-dimethylphenyl)-2-piperidincarboxamid (Bupivacain), (*S*)-1-Propyl-2',6'-dimethyl-2-piperidylcarboxyanilid (Ropivacain), 2-(Ethylpropylamino)-2',6'-butyroxylidid (Etidocain) und 1-(4-Butoxyphenyl)-3-piperidin-1-ylpropan-1-on (Dyclonin).

Noch mehr bevorzugt umfasst das Kohlensäure-Addukt (KA) mindestens ein Amin ausgewählt aus der Gruppe 4-Aminobenzoesäure-2-(*N*,*N*-diethylamino-)ethylester (Procain), 2-Diethylamino-*N*-(2,6-dimethylphenyl)acetamid (Lidocain) und (2-(Dimethylamino)ethyl)-4-(butylamino)benzoat (Tetracain).

Besonders bevorzugt umfasst das Kohlensäure-Addukt (KA) mindestens ein Amin (AM) ausgewählt aus der Gruppe 4-Aminobenzoesäure-2-(*N*,*N*-diethylamino-)ethylester (Procain) und 2-Diethylamino-*N*-(2,6-dimethylphenyl)acetamid (Lidocain).

Ganz besonders bevorzugt umfasst das Kohlensäure-Addukt (KA) 4-Aminobenzoesäure-2-(*N*,*N*-diethylamino-)ethylester (Procain) als Amin (AM).

Optional umfasst das Kohlensäure-Addukt (AM) mindestens ein Salz (S). Vorzugsweise umfasst das Kohlensäure-Addukt (AM) mindestens ein Salz (S). Vorzugsweise umfasst das Kohlensäure-Addukt (AM) mindestens ein Salz (S), wenn das Amin (AM) Procain oder Lidocain ist.

Bei dem mindestens einen Salz (S) handelt es sich vorzugsweise um ein Salz, das aus mindestens einem Kation ausgewählt aus Na⁺, K⁺, Li⁺, Mg²⁺, Zn²⁺, Fe²⁺, Fe³⁺ und Mn²⁺ und mindestens einem Anion ausgewählt aus Cl⁻, Br⁻, J⁻, F⁻, SO₄²⁻, SO₃²⁻, HSO₄⁻ HSO₃⁻,⁻HCO₃⁻, CO₃²⁻, PO₄³⁻, HPO₄²⁻, H₂PO₄⁻, SiO₄⁴⁻, AlO₂⁻, SiO₃⁻ und /oder [AlO₂)₁₂(SiO₂)₂]²⁻zusammengesetzt ist. Mehr bevorzugt ist das Kation ausgewählt aus Na⁺ und das Anion ausgewählt aus Cl⁻ oder Br.⁻ Das Salz kann beispielsweise, durch eine Säure-Base-Reaktion der Base (BA), bei Durchführung von Schritt b) mit der an das Amin (AM) addierten Säure gebildet werden, falls ein Säureaddtionssalz des Amins (AM) eingesetzt wird. Das Salz (S) kann auch direkt in einem der Schritte a), b) und/oder c) zugesetzt werden. Der direkte Zusatz des Salzes (S) ist bevorzugt, falls das Amin (AM) nicht in der Salzform eingesetzt und/oder der Schritt b) nicht durchgeführt wird.

Vorzugsweise bleibt das Kohlensäure-Addukt (KA) bei Lagerung bei einer Temperatur von 2 bis 10 °C für mindestens 12 Monate, mehr bevorzugt für mindestens 13 Monate, noch mehr bevorzugt für mindestens 20 Monate, besonders bevorzugt für mindestens 23 Monate, und ganz besonders bevorzugt für mindestens 27 Monate stabil.

Das Kohlensäure-Addukt (KA) gilt nicht mehr als stabil, wenn mit IR-Spektroskopie, insbesondere im Feststoff des Kohlensäure-Adduktes (KA), die spezifischen Banden des mindestens einen Amins (AM) detektiert werden können. Die spezifischen IR-Banden des Amins sind diejenigen Banden, die auch bei der IR-spektroskopischen Untersuchung des reinen Amins (AM) detektiert werden. Solange das Amin (AM) gebunden im stabilen Kohlensäure-Addukt (KA) vorliegt, werden die spezifischen IR-Banden des Amins (AM) nicht detektiert.

Der Verlust der Stabilität kann in einzelnen Ausführungsformen auch mit einer Erhöhung des pH-Wertes, oder mit der Messung von zwei Schmelz-/Zersetzungsbereichen, d.h. einem Bereich der dem Amin (KA) entspricht und einem Bereich der dem Kohlensäure-Addukt (KA) entspricht einhergehen. Bei dem Kohlensäure-Addukt, das durch zumindest teilweise Zersetzung in das Amin (AM) und CO₂ und/oder Wasser, instabil geworden ist, kann es auch zu einer Änderung des Lösungsverhaltens kommen. Das instabil gewordene Kohlensäure-Addukt (KA) kann sich als schwerlöslicher erweisen oder zumindest teilweise unvollständig gelöst werden.

Das Kohlensäure-Addukt (KA) ist herstellbar nach einem Verfahren umfassend die Schritte a), optional b), c), d) und e).

In Schritt a) wird eine Lösung (A) bereitgestellt, die mindestens ein Lösungsmittel und in dem mindestens einen Lösungsmittel gelöstes CO₂ umfasst.

Die Lösung (A) umfasst mindestens ein Lösungsmittel und CO₂ in gelöster Form. Unter CO₂ wird im Rahmen dieser Erfindung Kohlendioxid verstanden. Unter CO₂ in gelöster Form werden im Rahmen dieser Erfindung alle Formen des CO₂ verstanden, die dieses beim Lösen eingeht. So ist beispielweise für wässrige Lösungen bekannt, dass das gelöste CO₂ in der Lösung unter anderem im Gleichgewicht als CO₂, als Kohlensäure, als einfach oder zweifach deprotonierte Kohlensäure, das heißt als Hydrogencarbonat oder Carbonat vorliegen kann.

Die Lösung (A) wird erhalten, in dem CO₂ in das mindestens eine Lösungsmittel eingebracht wird. Das CO₂ kann in allen dem Fachmann bekannten und geeigneten Formen und in das Lösungsmittel eingebracht werden. Vorzugsweise wird gasförmiges oder gefrorenes CO₂ in Form von Trockeneis, mehr bevorzugt gasförmiges CO₂ in das Lösungsmittel eingebracht. Das CO₂ kann auch unter Druck eingebracht werden, insbesondere wenn gasförmiges CO₂ in die Lösung eingebracht wird. Einbringen unter Druck bedeutet in diesem Zusammenhang, dass ein Druck größer dem Atmosphärendruck, vorzugsweise größer 1,01325 bar, verwendet wird. Zu diesem Zweck kann das Einbringen des CO₂ in das Lösungsmittel in einem Behälter erfolgen, der das Lösungsmittel derartig von der Umgebung isoliert, dass in dem Behälter ein Druck erzeugt werden kann, insbesondere über das Zuleiten des CO₂, der über dem atmosphärischen Druck, vorzugsweise über 1,01325 bar liegt. Das Einbringen des CO₂ in die Lösung, insbesondere in gasförmiger Form, kann in einem Schritt oder in Intervallen erfolgen.

Der Fachmann kann in Schritt a) jedes geeignete Lösungsmittel einsetzen. Vorzugsweise wird als Lösungsmittel ein polar-protisches Lösungsmittel eingesetzt, mehr bevorzugt ist das Lösungsmittel Wasser. Das Lösungsmittel kann je nach beabsichtigtem Verwendungszweck des Kohlensäure-Addukts (KA) in verschiedenen Reinheitsgraden eingesetzt werden. Beispielsweise kann Wasser mit dem Reinheitsgrad "Aqua ad iniectabilia" verwendet werden, falls das Kohlensäure-Addukt (KA) für pharmazeutisch-medizinische Zwecke eingesetzt werden soll.

Schritt a) kann den Teilschritt a1) umfassen, wobei das Lösungsmittel, vorzugsweise vor dem Einbringen des CO₂, auf 3 bis 8 °C, vorzugsweise auf 5 °C gekühlt wird. Das Kühlen kann mittels allen dem Fachmann bekannten und als geeignet identifizierten Methoden erfolgen. Beispielsweise kann das Kühlen durch Verwahren des Lösungsmittels für eine ausreichend lange Zeit in einem Kühlschrank erfolgen, bis das Lösungsmittel die Zieltemperatur aufweist. Ebenso kann beispielsweise eine externe Kühlung verwendet werden.

Schritt a) kann den Teilschritt a2) umfassen, wobei das CO₂ in das Lösungsmittel eingebracht wird, vorzugsweise bis zum Erreichen einer Sättigungskonzentration von 3 bis 10 g/l, mehr bevorzugt bis zu einer Sättigungskonzentration von 4,5 bis 7,5 g/l bezogen auf das Gesamtvolumen der Lösung. Vorzugsweise beträgt der pH-Wert der Lösung nach der Sättigung mit CO₂ ≤ 3,0 bis ≤ 6,0, noch mehr bevorzugt ≤ 4,3 bis ≤ 4,8. Vorzugsweise wird das CO₂ in Teilschritt a2) unter Druck gelöst, wobei der Druck 1,5 bis 10 bar, mehr bevorzugt 1,9 bis 7 bar, noch mehr bevorzugt 2 bis 5 bar beträgt.

Schritt a) kann den Teilschritt a3) umfassen, wobei die vorzugsweise in Teilschritt a2) erhaltene Lösung (A) bei 1 bis 10 °C vorzugsweise für mindestens 30 min, mehr bevorzugt für mindestens 50 min, noch mehr bevorzugt für mindestens 60 min; bis höchstens 5d (120h) gelagert wird. Vorzugsweise wird die vorzugsweise in Teilschritt a2) erhaltene Lösung (A) bei 3 bis 8 °C für mindestens 30 min, mehr bevorzugt für mindestens 50 min, noch mehr bevorzugt für mindestens 60 min; bis höchstens 5d (120h) gelagert.

Vorzugsweise umfasst Schritt a) alle Teilschritte a1), a2) und a3).

Vorzugsweise werden die Teilschritte a1), a2) und a3) in der Reihenfolge a2) folgt auf a1) und a3) folgt auf a2) durchgeführt.

Optional kann der Schritt b) durchgeführt werden, wobei die Base (BA), die nicht dem Amin (AM) entspricht, in der Lösung (A), unter Erhalt der Lösung (A1) aufgelöst wird. Vorzugsweise ist die Base (BA) ein Hydrogencarbonat oder ein Carbonat, mehr bevorzugt ein Hydrogencarbonat, noch mehr bevorzugt Natriumhydrogencarbonat.

In Schritt c) wird das mindestens eine Amin (AM) in der Lösung (A) oder (A1) unter Erhalt der Lösung (B) gelöst.

Das mindestens eine Amin (AM), wie vorstehend definiert, kann in Schritt c) sowohl in neutraler Form als auch in Form eines Salzes eingesetzt werden. Optional kann das mindestens eine Amin (AM) auch als Mischung der neutralen Form des Amins (AM) mit der Salzform des Amins (AM) eingesetzt werden. Daher kann das mindestens eine Amin (AM), das neutrale Amin (AM) und/oder die Salzform des mindestens einen Amins (AM) umfassen. Vorzugsweise handelt es sich bei der Salzform des mindestens einen Amins (AM) um ein Säureadditionssalz, vorzugsweise ist das Säureadditionsalz ein Hydrochlorid, Hydrobromid, Hydroiodid, Hydrogensulfat, Hydrogensulfit, Hydrogenphosphat, Hydromesylat, Hydrotosylat, Hydroacetat, Hydroformiat, Hydropropanoat, Hydromalonat, Hydrosuccinat, Hydrofumarat, Hydroxalat, Hydrotartrat, Hydrocitrat, Hydromaleat, mehr bevorzugt ein Hydrochlorid oder Hydrobromid, noch mehr bevorzugt ein Hydrochlorid des mindestens einen Amins (AM).

Vorzugsweise beträgt die Konzentration des Amins (AM) in Lösung (B) 0,01 bis 0,25 g/ml, vorzugsweise 0,03 bis 0,20 g/ml, mehr bevorzugt 0,08 bis 0,15 g/ml.

Schritt c) kann den Teilschritt c2) umfassen, wobei das mindestens eine Amin (AM) in Lösung (A), oder bei Durchführung von Schritt b), in Lösung (A1) unter Erhalt der Lösung (B) aufgelöst wird.

In einer möglichen Ausführungsform beträgt das Verhältnis des Amins (AM) zur Base (BA), bei Durchführung von Schritt b), in Lösung (B) 2:1 bis 5:1, mehr bevorzugt 3:1 bis 4:1, noch mehr bevorzugt 3,23:1 bis 3,26:1 [g/g]].

In einer weiteren möglichen Ausführungsform beträgt das molare Verhältnis des Amins (AM) zu den Basenequivalenten der Base (BA), bei Durchführung von Schritt b), in Lösung (B) 0,8:1 bis 1,5:1, vorzugsweise 1,2:1, mehr bevorzugt 1:1. Basenequivalente bedeutet in diesem Zusammenhang, dass bei Verwendung einer einwertigen Base, wie zum Beispiel NaHCO₃ das molare Verhältnis der Base (BA) zum Amin (AM dem vorstehend angegebenen Verhältnis entspricht. Bei Verwendung einer zweiwertigen Base (BA), wie zum Beispiel Na₂CO₃, ist bezogen auf die Stoffmenge in Mol der Base (BA) relativ zur Verwendung einer einwertigen Base nur die Hälfte der Basenmenge erforderlich um die gleiche Menge an Basenequivalenten einzubringen. So werden zum Beispiel bei einem Verhältnis von 1:1, bei Verwendung von 10 mmol Amin (AM), 10 mmol NaHCO₃ benötigt, aber nur 5 mmol Na₂CO₃.

In einer weiteren Ausführungsform wird Schritt b) ausgeführt und in Teilschritt c1) das Amin (AM) in Form des Säureadditionssalzes zugesetzt, wobei das Amin (AM) mit der an ihm gebundenen Säure, in einer derartigen Menge zugesetzt wird, dass die an das Amin (AM) gebundene Säure, die Base (BA) soweit zu neutralisieren vermag, dass die Lösung (B) einen pH-Wert von 6 bis 8 annimmt.

Schritt c) kann den Teilschritt c2) umfassen, wobei Lösung (A) zur Lösung (B) unter Erhalt der Lösung (B1) gegeben wird.

Vorzugsweise beträgt die Konzentration des Amins (AM) in Lösung (B1) 0,01 bis 0,25 g/ml, vorzugsweise 0,03 bis 0,20 g/ml, mehr bevorzugt 0,08 bis 0,15 g/ml.

Schritt c) kann den Teilschritt c3) umfassen, wobei die Lösung (B) oder bei Durchführung von Teilschritt c2), die Lösung (B1) mit CO₂ angereichert wird. Vorzugsweise wird die Lösung (B) mit 2,5 g/l bis 9 g/l, mehr bevorzugt mit 5 bis 7,5 g/l CO₂ angereichert.

Schritt c) kann den Teilschritt c4) umfassen, wobei die Lösung (B) oder, bei Durchführung von Teilschritt c2), die Lösung (B1) bei 1 bis 10°C, vorzugsweise 3 bis 8 °C, für mindestens 1 h vorzugsweise 24 h bis 120 h, noch mehr bevorzugt 24 bis 72 h gelagert wird.

Schritt c) kann den Teilschritt c5) umfassen, wobei die Lösung (B) oder bei Durchführung von Teilschritt b2) die Lösung (B1) mit CO₂ auf eine Gesamtkonzentration von mindestens 6 g/l, vorzugsweise mindestens 10 g/l, mehr bevorzugt mindestens 12 g/l, noch mehr bevorzugt mindestens 14 g/l und ganz besonders bevorzugt mindestens 15 g/l angereichert wird. Vorzugsweise werden in Teilschritt c5) weitere 0,4 bis 4,7 g/l, mehr bevorzugt 1 bis 3,5 g/l CO₂ bis zum Erreichen der benötigten Gesamtkonzentration in die Lösung (B) oder (B1) eingebracht bzw. gelöst.

Der Begriff "Gesamtkonzentration" bezieht sich hier auf die Gesamtkonzentration an gelöstem CO₂ in der Lösung (B) oder (B1), einschließlich dem CO₂, das im Kohlensäure-Addukt (KA) gebunden ist. Die Gesamtkonzentration ergibt sich additiv aus der Gewichtszunahme der Lösung durch das zugeführte CO₂ in allen vorangegangenen Anreicherungsschritten a2) und/oder c3), soweit ausgeführt, und c5), ohne Berücksichtigung von CO₂, das optional in Form von Hydrogencarbonat oder Carbonat als Base (BA) der Lösung zugesetzt wird.

Das Anreichern der Lösung (B) oder der Lösung (B1) in Teilschritt c5) mit CO₂ auf die benötigte Gesamtkonzentration kann bei einem Druck von 2,5 bis 10 bar, vorzugsweise von 4 bis 10 bar, mehr bevorzugt von 5 bis 10 bar, noch mehr bevorzugt bei 6 bis 10 bar, ganz besonders bevorzugt bei 6,5 bis 10 bar durchgeführt werden. Vorzugsweise weist die Lösung (B) oder (B1), beim Anreichern mit CO₂ in Teilschritt c5) eine Temperatur von 3 bis 8 °C, mehr bevorzugt von 5°C auf.

Die Anreichung der Lösung (B) oder (B1) in den Teilschritten c3) und c5) kann auf dieselbe Weise erfolgen, wie für Schritt a) beschrieben.

Vorzugsweise beträgt der pH-Wert der Lösung (B) oder, bei Durchführung des Teilschritts c2), der Lösung (B1) nach der Durchführung von Schritt c5) ≤ 7,0.

Vorzugsweise umfasst Schritt c) alle Teilschritte c1), c2), c3), c4) und c5).

Vorzugsweise werden die Teilschritte c1), c2), c3), c4) und c5) in der Reihenfolge c2) folgt auf c1), c3) folgt auf c2), c4) folgt auf c3), c5) folgt auf c4) durchgeführt.

In Schritt d) wird die nach Abschluss des Schrittes c) erhaltene Lösung eingefroren. Vorzugsweise wird in Schritt d) die Lösung B) oder nach Durchführung des Teilschrittes c2), die Lösung (B1) eingefroren.

Die Lösung, vorzugsweise Lösung (B) oder (B1), die dem Schritt d) unterzogen wird, weist einen CO₂-Gehalt von mindestens 6 g/l, vorzugsweise mindestens 10 g/l, mehr bevorzugt mindestens 12 g/l, noch mehr bevorzugt mindestens 14 g/l und ganz besonders bevorzugt mindestens 15 g/l auf.

Vorzugsweise wird die nach Abschluss des Schrittes c) erhaltene Lösung, vorzugsweise Lösung (B) oder (B1), bei -100 °C bis -20 °C, mehr bevorzugt bei -90 °C bis -30 °C, noch mehr bevorzugt bei -80 bis -40 °C und ganz besonders bevorzugt bei -70 bis -50 °C eingefroren.

Das Einfrieren der nach Abschluss des Schrittes c) erhaltenen Lösung, vorzugsweise Lösung (B) oder (B1), kann grundsätzlich nach allen dem Fachmann bekannten und als geeignet identifizierten Methoden erfolgen. Beispielsweise kann das Einfrieren durch das Überführen der in Schritt c) erhaltenen Lösung in ein geeignetes Gefäß, erfolgen, dass in ein Kühlmedium eintaucht. Vorzugsweise hat das Gefäß eine Kolbenform. Vorzugsweise taucht das Gefäß, in dem sich die in Schritt c) erhaltene Lösung befindet in einem Winkel von 40 ° in das Kühlmedium ein. Das Kühlmedium kann beispielsweise aus einem Lösungsmittel wie Methanol, Ethanol oder Aceton bestehen, das durch Zugabe von Trockeneis, oder geeignete Kühlapparaturen, wie Kryostaten auf die gewünschte Temperatur gebracht wird.

Vorzugsweise erfolgt das Einfrieren bei Atmosphärendruck, mehr bevorzugt bei 1,01325 bar.

Vorzugsweise wird die nach Abschluss des Schrittes c) erhaltene Lösung, vorzugsweise Lösung (B) oder (B1), innerhalb von 0,3 bis 60 Minuten, mehr bevorzugt innerhalb von 1 bis 30 Minuten, noch mehr bevorzugt innerhalb von 1,1 bis 10 Minuten, besonders bevorzugt innerhalb von 1,5 bis 5 Minuten eingefroren.

Die nach Abschluss des Schrittes c) erhaltene Lösung, vorzugsweise Lösung (B) oder (B1), wird vorzugsweise mit einer Kühlrate von 10 bis 100 K/min, mehr bevorzugt mit 20 bis 80 K/min, noch mehr bevorzugt mit 30 bis 70 K/min und besonders bevorzugt mit 40 bis 60 K/min eingefroren.

Vorzugsweise wird das Gefäß in dem sich die nach Abschluss des Schrittes c) erhaltene Lösung, vorzugsweise Lösung (B) oder (B1), während des Einfriervorgangs befindet, im Kühlmedium, mit 10 bis 1000 rpm, mehr bevorzugt mit 50 bis 600 rpm, noch mehr bevorzugt mit 100 bis 400 rpm und besonders bevorzugt mit 200 bis 300 rpm rotiert.

Das Einfrieren kann nach dem Shell-Freeze-Verfahren erfolgen.

In Schritt e) wird die in Schritt d) eingefrorene Lösung, vorzugsweise Lösung (B) oder (B1), bei -100 bis 0 °C für nicht länger als 4 Tage gelagert.

Vorzugsweise wird die in Schritt d) eingefrorene Lösung in Schritt e), vorzugsweise Lösung (B) oder (B1), für 1,5 bis 4 Tage, mehr bevorzugt für 2,5 bis 4 Tage gelagert.

Vorzugsweise wird die in Schritt d) eingefrorene Lösung in Schritt e), vorzugsweise Lösung (B) oder (B1) bei -50 bis 0°C, mehr bevorzugt bei -30 bis -5 °C, noch mehr bevorzugt bei -25 bis -10 °C, besonders bevorzugt bei -20 bis -15 °C gelagert.

Die Lagerung kann bei der definierten Temperatur grundsätzlich in jeder dem Fachmann bekannten Kühleinrichtung erfolgen. Beispielsweise kann die Lagerung in einem Tiefkühlschrank oder einem Tiefkühlraum durchgeführt werden.

Das Verfahren nach dem das Kohlensäure-Addukt (KA) herstellbar ist, kann einen weiteren Schritt f) umfassen, der nach Schritt e) durchgeführt wird. Dabei wird in Schritt f) die in Schritt e) gelagerte Lösung, vorzugsweise Lösung (B) oder (B1), getrocknet, unter Erhalt von getrocknetem Kohlensäure-Addukt (KA).

Vorzugsweise wird in Schritt f) das Wasser aus der in Schritt e) gelagerten Lösung, vorzugsweise Lösung (B) oder (B1) bis zu einem Restgehalt von < 0,8 Gew.-%, mehr bevorzugt bis zu einem Restgehalt von < 0,1 Gew.-% bezogen auf das Gesamtgewicht des getrockneten Kohlensäure-Adduktes (KA) entfernt.

Vorzugsweise wird in Schritt f) nicht im Kohlensäure-Addukt (KA) gebundenes CO₂ aus der in Schritt e) gelagerten Lösung, vorzugsweise (B) oder (B1), bis zu einem Restgehalt von < 0,8 Gew.-%, mehr bevorzugt bis zu einem Restgehalt von < 0,1 Gew.-% bezogen auf das Gesamtgewicht des getrockneten Kohlensäure-Adduktes (KA) entfernt.

Die Trocknung kann mit allem dem Fachmann bekannten und als geeignet identifizierten Methoden durchgeführt werden. Vorzugsweise erfolgt die Trocknung mittels Gefriertrocknung, auch Lyophilisation genannt. Der Schritt d) stellt im Falle der Anwendung des Gefriertrocknungsverfahrens, den Einfrierschritt und Schritt e) den Reifungsschritt dar.

Vorzugsweise beträgt der Druck während des Trocknens 0,01 bis 30 mbar, vorzugsweise 0,02 bis 20 mbar, mehr bevorzugt 0,03 bis 10 mbar, noch mehr bevorzugt 0,03 bis 0,5 mbar und ganz besonders bevorzugt 0,05 bis 0,1 mbar. Vorzugsweise wird der Druck während des gesamten Trocknungsvorgangs beibehalten. Vorzugsweise wird der vorstehend definierte Druck während des Trocknens innerhalb von 7h, mehr bevorzugt innerhalb von 5h und besonders bevorzugt innerhalb von 4 h ab Evakuierungsbeginn erreicht.

Den Endpunkt der Trocknung kann der Fachmann aus den Temperaturverlaufsaufzeichnungen ermitteln. Vorzugsweise beträgt die Gesamttrocknungszeit in Schritt f) 10 bis 60 h, mehr bevorzugt 30 bis 55 h, besonders bevorzugt 41 bis 52 h. Die Gesamttrocknungszeit ist definiert als die Zeitspanne zwischen dem Abschluss der Lagerung in Schritt e) und der Beendigung der Trocknung in Schritt f).

Vorzugsweise beträgt die Temperatur während der gesamten Trocknung in Schritt f) 0 bis 20 °C, vorzugsweise 4 bis 18 °C, mehr bevorzugt 8 bis 16 °C.

Das vorstehend beschriebene Verfahren zur Herstellung des Kohlensäure-Adduktes (KA) ist ein weiterer Aspekt der Erfindung.

In einer weiteren Ausführungsform der Erfindung umfasst das Kohlensäure-Addukt (KA), Procain als Amin (AM), Kohlensäure und mindestens ein Salz (S), wobei der Zersetzungspunkt 65 bis 95 °C, vorzugsweise 70 bis 90 °C, mehr bevorzugt 85 bis 90 °C beträgt oder Lidocain als Amin (AM), Kohlensäure und mindestens ein Salz (S), wobei der Zersetzungspunkt 20 bis 45 °C, vorzugsweise 25 bis 45 °C, mehr bevorzugt 30 bis 45 °C beträgt und/oder das Kohlensäure-Addukt (KA) mindestens ein Amin (AM), Kohlensäure und optional mindestens ein Salz (S) umfasst und bei Lagerung bei einer Temperatur von 2 bis 10 °C für mindestens 12 Monate, vorzugsweise für mindestens 13 Monate, mehr bevorzugt für mindestens 20 Monate, noch mehr bevorzugt für mindestens 23 Monate, besonders bevorzugt für mindestens 27 Monate stabil bleibt.

Ein weiterer Aspekt der Erfindung ist eine pharmazeutische Zubereitung (PZ) umfassend das Kohlensäure-Addukt (KA).

Unter der pharmazeutischen Zubereitung (PZ) im Rahmen dieser Erfindung wird grundsätzlich eine Zusammensetzung verstanden, die das Kohlensäure-Addukt (KA) umfasst und darüber hinaus, weitere Hilfsstoffe oder Zusätze umfassen kann, die für eine pharmazeutisch-medizinische Verwendung geeignet sind.

Außerdem kann die pharmazeutische Zubereitung (PZ) weitere Basen umfassen, die nicht dem Amin (AM) entsprechen und verschieden von der Base (BA) sein können. Der Fachmann kann die Additive grundsätzlich je nach gewünschtem Verwendungszweck auswählen. Er wird dabei die gewünschte Applikationsform berücksichtigen.

Die pharmazeutische Zubereitung (PZ) kann grundsätzlich in jeder geeigneten Darreichungsform vorliegen. So kann die pharmazeutische Zubereitung (PZ) beispielsweise in Kapselform, als Tablette, als Lösung, als Salbe, Creme, als Gel, als Paste, Umschlagpaste oder wirkstoffhaltiges Pflaster vorliegen.

Die pharmazeutische Zubereitung (PZ) kann grundsätzlich in jeder geeigneten Applikationsform appliziert werden. Der Fachmann wird eine geeignete Darreichungsform entsprechend der beabsichtigten Applikationsform auswählen. Beispielsweise kann die pharmazeutische Zubereitung (PZ) oral, inhalativ, per Injektion, als Pflaster, kutan umfassend mindestens die dermale Anwendung, die Anwendung am Auge, die nasale Anwendung, die rektale Anwendung und die vaginale Anwendung; verabreicht werden.

Bei der Zubereitung der pharmazeutischen Zubereitung (PZ) kann sich der Fachmann grundsätzlich der im Stand der Technik bekannten Methoden bedienen.

Vorzugsweise beträgt die Temperatur der Mischung aus dem Kohlensäure-Addukt (KA) und den verwendeten Hilfsstoffen und gegebenenfalls weiteren Basen während der Zubereitung der pharmazeutischen Zubereitung (PZ) weniger als 60 °C, vorzugsweise weniger als 50 °C, mehr bevorzugt 0 bis 50 °C.

Bei der Zubereitung der pharmazeutischen Zubereitung (PZ), vorzugsweise in Salbenform, kann auch Dispergieren, vorzugsweise mittels eines Salbenbereiters, zum Einsatz kommen. Hierbei wird vorzugsweise eine Drehzahl von < 2000 rpm verwendet.

Das Kohlensäure-Addukt (KA) kann vor der Verarbeitung zu den nachfolgend beschriebenen oralen Darreichungsformen wie Tabletten oder Kapseln oder halbfesten Darreichungsformen, alleine oder in der Anwesenheit weiterer Hilfsstoffe oder Basen zu Pulver verrieben werden. Der Fachmann kann sich grundsätzlich der für den jeweiligen Zweck geeigneten und bekannten technischen Mittel bedienen. So können für Zerreibungsschritte beispielsweise Mörser oder ähnlich geeignete Geräte eingesetzt werden. Vorzugsweise wird bei den Verreibungsschritten ein technisches Hilfsmittel eingesetzt, dass die mechanische Belastung des Kohlensäure-Adduktes möglichst gering hält. Vorzugsweise erfolgt das Zerreiben mit einem Mörser.

Das so erhaltene Pulver kann dann zum Beispiel zu Tabletten gepresst oder in handelsübliche Kapseln abgefüllt oder mit geeigneten Hilfsstoffen vermischt und zu halbfesten Darreichungsformen verarbeitet werden.

Eine Ausführungsform der pharmazeutischen Zubereitung (PZ) betrifft eine pharmazeutische Zubereitung (PZ), die das Kohlensäure-Addukt (KA) umfasst, und oral appliziert wird. In dieser Ausführungsform wird die pharmazeutische Zubereitung (PZ) vorzugsweise in Kapseln, mehr bevorzugt in Hartgelatine oder Cellulose-Kapseln, besonders bevorzugt in Hartgelatine Kapseln verabreicht. Ebenso kann die pharmazeutische Zubereitung (PZ) in dieser Ausführungsform in Tablettenform appliziert werden.

Vorzugsweise umfasst die pharmazeutische Zubereitung (PZ) in dieser Ausführungsform mindestens ein Hilfsstoff (H), vorzugsweise ausgewählt aus Stärke, insbesondere Maisstärke und/oder Reisstärke, Dextran, Celluloseester und SiO₂.

Zudem kann die pharmazeutische Zubereitung (PZ) in dieser Ausführungsform mindestens eine Base (BA1) umfassen, die nicht dem Amin (AM) entspricht und identisch oder verschieden von der Base (BA) ist. Vorzugsweise ist die Base (BA1) ausgewählt aus NaHCO₃ oder KHCO₃, mehr bevorzugt NaHCO₃.

Vorzugsweise gelten auch für diese Ausführungsform die vorstehend gemachten allgemeinen Angaben zur pharmazeutischen Zubereitung (PZ), insbesondere auch für die Zubereitung der pharmazeutischen Zubereitung (PZ) soweit für diese Ausführungsform technisch anwendbar.

Vorzugsweise umfasst die pharmazeutische Zubereitung (PZ) in dieser Ausführungsform
a) 1 bis 99 Gew.-%, mehr bevorzugt 15 bis 95 Gew.-% des Kohlensäure-Addukts (KA),
b) 0 bis 60 Gew.-%, mehr bevorzugt 3 bis 50 Gew.-% der Base (BA1) und 1 bis 90 Gew.-%, mehr bevorzugt 2 bis 75 Gew.-% des Hilfsstoffs (H) bezogen auf das Gesamtgewicht der pharmazeutischen Zubereitung.

Weiterhin umfasst die Erfindung ein Verfahren zur Herstellung der pharmazeutischen Zubereitung (PZ) in der vorstehend genannten Ausführungsform zur oralen Applikation, umfassend die Schritte:
a) Bereitstellung einer Mischung umfassend das Kohlensäure-Addukt (KA) und optional mindesten eine Base (BA1) und/oder mindestens einen Hilfsstoff (H),
b) Verreiben der Mischung zu Pulver,
c) Verarbeitung des Pulvers in eine Darreichungsform zur oralen Applikation,
wobei optional
i) der mindestens eine Hilfsstoff (H) ausgewählt ist aus Stärke, insbesondere. Maisstärke und/oder Reisstärke, Dextran, Celluloseester und SiO₂ und/oder
ii) die Base (BA1) ausgewählt ist aus NaHCO₃ oder KHCO₃, mehr bevorzugt NaHCO₃ und/oder
iii) die Darreichungsform zur oralen Applikation eine Tablette ist und/oder
iv) die Darreichungsform zur oralen Applikation eine Kapsel, vorzugsweise eine Hartgelatine- oder Cellulose- Kapsel, mehr bevorzugt eine Hartgelatine-Kapsel ist und/oder
v) vom Kohlensäure-Addukt (KA) 1 bis 99 Gew.-%, vorzugsweise 15 bis 95 Gew.-% bezogen auf das Gesamtgewicht der pharmazeutischen Zubereitung (PZ) eingesetzt werden und/oder
vi) von der Base (BA1) 0 bis 60 Gew.-%, vorzugsweise 3 bis 50 Gew.-% bezogen auf das Gesamtgewicht der pharmazeutischen Zubereitung (PZ) eingesetzt werden und/oder
vii) von dem Hilfsstoff (H) 1 bis 90 Gew.-%, vorzugsweise 2 bis 75 Gew. -% bezogen auf das Gesamtgewicht der pharmazeutischen Zubereitung (PZ) eingesetzt werden.

Weiterhin umfasst die Erfindung ein Kit zur Herstellung der vorstehend genannten Ausführungsform der pharmazeutischen Zubereitung (PZ) zur oralen Applikation, umfassend
a) das Kohlensäure-Addukt (KA),
b) optional die Base (BA1) und
c) den Hilfsstoff (H).

Eine weitere Ausführungsform der pharmazeutischen Zubereitung (PZ) betrifft eine halbfeste pharmazeutische Zubereitung (PZ), die das Kohlensäure-Addukt (KA) umfasst, und kutan appliziert wird. Die pharmazeutische Zubereitung (PZ) in dieser Ausführungsform kann beispielsweise in Salbenform, als Creme, als Gel, als Paste, Umschlagspaste oder wirkstoffhaltiges Pflaster appliziert werden.

Vorzugsweise gelten auch für diese Ausführungsform die vorstehend gemachten allgemeinen Angaben zur pharmazeutischen Zubereitung (PZ), insbesondere auch für die Herstellung der pharmazeutischen Zubereitung (PZ) soweit für diese Ausführungsform technisch anwendbar.

Die pharmazeutische Zubereitung (PZ) umfasst in dieser Ausführungsform vorzugsweise mindestens ein Hilfsstoff (H1) ausgewählt aus Paraffinen, insbesondere dick- und dünnflüssigen Paraffinen, Wollwachs, Wollwachsalkoholen, hydrophobes Basisgel, pflanzliche Öle, tierische Fette, synthetische Glyceride, flüssige Polyalkylsiloxane, Wachse, Vaseline und Stärke, insbesondere Maisstärke, vorzugsweise Vaseline.

Unter dickflüssigen Paraffinen (*Paraffinum subliquidum*) werden Paraffine verstanden, die eine Viskosität von 110 bis 230 mPas aufweisen, während dünnflüssige Paraffine (*Paraffinum perliquidum*) eine Viskosität von 25 bis 80 mPas aufweisen.

Vorzugsweise umfasst die pharmazeutische Zubereitung (PZ) in dieser Ausführungsform:
a) 0,1 bis 40 Gew.-%, vorzugsweise 0,4 bis 10 Gew.-% des Kohlensäure-Addukts (KA) und
b) 60 bis 99,9 Gew. -%, vorzugsweise 80 bis 96 Gew.-% des Hilfsstoffs (H1) bezogen auf die Gesamtmenge der pharmazeutischen Zubereitung (PZ) umfasst.

Außerdem betrifft die Erfindung ein Verfahren zur Herstellung der halbfesten pharmazeutischen Zubereitung (PZ) gemäß der vorstehend beschriebenen Ausführungsform, umfassend die Schritte
a) Zerreiben des Kohlenstoff-Adduktes (KA) zu Pulver,
b) Vermischen des Kohlenstoff-Adduktes (KA)-Pulvers aus Schritt a) mit mindestens einem Hilfsstoff (H1),
wobei optional
i) der mindestens eine Hilfsstoff (H1) ausgewählt ist aus Paraffine, insbesondere dick- und dünnflüssige Paraffine, Wollwachs, Wollwachsalkohole, hydrophobes Basisgel, pflanzliche Öle, tierische Fette, synthetische Glyceride, flüssige Polyalkylsiloxane, Wachse, Vaseline, Stärke, insbesondere Maisstärke, vorzugsweise Vaseline und/oder
ii) die Temperatur in Schritt a) und b) weniger als 60 °C, vorzugsweise weniger als 50 °C, mehr bevorzugt 20 bis 50 °C beträgt und/oder
iii) bei Anwendung von Dispergierern und/oder Salbenbereitern eine Drehzahl von < 2000 rpm verwendet wird und/oder
iv) 0,1 bis 40 Gew.-%, vorzugsweise 0,4 bis 10 Gew.-% des Kohlensäure-Addukts (KA) bezogen auf die Gesamtmenge der pharmazeutischen Zubereitung eingesetzt werden und/oder
v) 60 bis 99,9 Gew. -%, vorzugsweise 80 bis 96 Gew.-% des Hilfsstoffs (H1) bezogen auf die Gesamtmenge der pharmazeutischen Zubereitung (PZ) eingesetzt werden.

Weiterhin umfasst die Erfindung ein Kit zur Herstellung der vorstehend genannten Ausführungsform, umfassend
a) das Kohlensäure-Addukt (KA) und
b) den Hilfsstoff (H1).

Eine weitere Ausführungsform der pharmazeutischen Zubereitung (PZ), die das Kohlensäure-Addukt (KA) umfasst, betrifft eine pharmazeutische Zubereitung, die parenteral, nasal und/oder inhalativ appliziert wird.

Vorzugsweise gelten auch für diese Ausführungsform die vorstehend gemachten allgemeinen Angaben zur pharmazeutischen Zubereitung (PZ), insbesondere auch für die Zubereitung der pharmazeutischen Zubereitung (PZ) soweit für diese Ausführungsform technisch anwendbar.

Vorzugsweise liegt die pharmazeutische Zubereitung (PZ) in dieser Ausführungsform als Lösung (A2) vor, umfassend das Kohlensäure-Addukt (KA), gelöstes CO₂ und mindestens ein Hilfsstoff (H2).

Der Hilfsstoff (H2) ist vorzugsweise ausgewählt aus einem Alkalihalogenid oder Erdalkalihalogenid, mehr bevorzugt NaCl und MgCl₂, noch mehr bevorzugt NaCl. Der Hilfsstoff (H2) kann identisch mit dem Salz (S) sein. Mengenangaben bezogen auf den Hilfsstoff (H2), soweit dieser in einzelnen Ausführungsformen identisch mit dem Salz (S) ist, beziehen sich im Rahmen dieser Erfindung auf zusätzliche Mengen des Hilfsstoffes (H2), die nicht in Form des Salzes (S) als Teil des Kohlensäure-Adduktes (KA) in die pharmazeutische Zubereitung (PZ) eingebracht wurden.

Vorzugsweise wird die Lösung (A2) durch Einbringen von CO₂ in ein Lösungsmittel erhalten. Vorzugsweise ist das Lösungsmittel Wasser. Vorzugsweise wird das CO₂ zur Herstellung der Lösung (A2) bei einer Temperatur von 0 bis 8 °C, mehr bevorzugt bei 0 bis 5 °C in das Lösungsmittel eingebracht. Das CO₂ kann in Form des Gases als auch in fester Form, etwa als Trockeneis in das Lösungsmittel eingebracht werden. Vorzugsweise wird das CO₂ in Form des Gases in das Lösungsmittel eingebracht Das CO₂ kann auch unter Druck, bis zum Erreichen der gewünschten Konzentration in die Lösung eingebracht werden, wie vorstehend etwa für Teilschritt a2) beschrieben.

Vorzugsweise wird das CO₂ zur Herstellung der Lösung (A2), bis zu einer Konzentration von mindestens 3 g/l, mehr bevorzugt bis 4 g/l, noch mehr bevorzugt 4 g/l bis 8 g/l in das Lösungsmittel eingebracht.

Vorzugsweise umfasst die pharmazeutische Zubereitung (PZ) in dieser Ausführungsform, insofern sie durch Auflösen des Kohlensäure-Adduktes (KA) in der Lösung (A2) erhalten wird,
a) 0,05 bis 100 mg/ml, mehr bevorzugt 0,08 bis 50 mg/ml des Kohlensäure-Adduktes (KA) und
b) 0 bis 20 mg/ml, mehr bevorzugt 3 bis 10 mg/ml des Hilfsstoffs (H2) bezogen jeweils auf das Gesamtvolumen der pharmazeutischen Zubereitung (PZ).

Die Erfindung betrifft ebenfalls ein Verfahren zur Herstellung der vorstehend beschriebenen Ausführungsform der pharmazeutischen Zubereitung zur parenteralen, nasalen und/oder inhalativen Applikation umfassend die Schritte:
a) Bereitstellung einer Lösung (A2),
b) Auflösen des Kohlensäure-Addukts (KA) in der Lösung (A2)
wobei optional
i) die Lösung (A2) vorzugsweise durch Einbringen von CO₂ in ein Lösungsmittel erhalten wird und/oder
ii) das Lösungsmittel der Lösung (A2) Wasser ist und/oder
iii) CO₂ zur Herstellung der Lösung (A2) bei einer Temperatur von 0 bis 8 °C, mehr bevorzugt bei 0 bis 5 °C in das Lösungsmittel eingebracht wird und/oder
iv) CO₂ in das Lösungsmittel der Lösung (A2) bis zu einer Konzentration von mindestens 3 g /I, vorzugsweise mindestens 4 g/l, noch mehr bevorzugt mindestens 4 g/l bis 8 g/l eingebracht wird und/oder
v) das Verfahren den weiteren Schritt c) Auflösen eines Hilfsstoffes (H2) in der Lösung (A2) umfasst und/oder
vi) bei Durchführung des Schrittes c) der Hilfsstoff (H2) ausgewählt ist aus einem Alkalihalogenid oder Erdalkalihalogenid, mehr bevorzugt aus NaCl und MgCl₂, noch mehr bevorzugt aus NaCl und/oder
vii) bei Durchführung des Schrittes c) 0 bis 20 mg/ml, vorzugsweise 3 bis 10 mg/ml des Hilfsstoffs (H2) bezogen auf das Gesamtvolumen der pharmazeutischen Zubereitung in Schritt c) in Lösung (A2) aufgelöst werden und/oder
viii) die Temperatur der Lösung (A2) in Schritt b) und/oder Schritt c) 0 bis 8 °C, mehr bevorzugt 0 bis 5 °C beträgt und/oder
ix) in Schritt b) 0,05 bis 100 mg/ml, vorzugsweise 0,08 bis 50 mg/ml, des Kohlensäure-Addukts (KA) in der Lösung (A2) gelöst werden.

Ein weiterer Aspekt der Erfindung ist ein Kit zur Herstellung der vorstehend beschriebenen pharmazeutischen Zubereitung umfassend:
a) das Kohlensäure-Addukt (KA),
b) die Lösung (A2), umfassend Wasser als Lösungsmittel und CO₂, vorzugsweise in einer Konzentration von mindestens 3 g/l, vorzugsweise mindestens 4 g/l, noch mehr bevorzugt mindestens 4 g/l bis 8 g/l und
c) den Hilfsstoff (H2), vorzugsweise ausgewählt aus einem Alkalihalogenid oder Erdalkalihalogenid, mehr bevorzugt aus NaCl und MgCl₂, noch mehr bevorzugt aus NaCl.

Die Erfindung umfasst außerdem auch die Zubereitung der pharmazeutischen Zubereitung (PZ), insbesondere auch der Ausführungsformen der pharmazeutischen Zubereitung (PZ), wie vorstehend beschrieben.

Ein weiterer Aspekt der Erfindung ist die Verwendung des Kohlensäure-Adduktes (KA) und der pharmazeutischen Zubereitung (PZ) sowie der vorstehend einzeln beschriebenen Ausführungsformen der pharmazeutischen Zubereitung (PZ) in einem Verfahren zur Anästhesie, zur Analgesie, zur begleitenden Behandlung von Krebs, zur Entzündungshemmung, zur Unterstützung der Wundheilung, insbesondere bei Verbrennungen, offenen Wunden und Narben, zur Behandlung von neurogenen Entzündungen wie Multiple Sklerose, MMN (Multifokale motorische Neuropathie), zur Behandlung von Sinusitis, zur Behandlung von Asthma, zur Behandlung von Rheumatischer Arthritis, zur Behandlung von Alzheimer, zur Behandlung von Demenz, zur Unterstützung der Rekonvaleszenz und zur Unterstützung des Anti-aging, zur Behandlung des Burn-out-Syndroms, zur Behandlung von Arthrose, zur Behandlung von Polyarthritis, zur Behandlung von Schmerzsyndrom und allgemeinen Schmerzen, zur prä- und postoperativen Behandlung (auch bei Knochenbrüchen), zur Präventiv- und Rehabilitationsmedizin, zur Behandlung von Zosterneuralgie, zur begleitenden Behandlung von Zosterneuralgie, zur Behandlung von Erkrankungen der Bauchorgane, wie Leber, Galle, Bauchspeicheldrüse, Darm, zur Behandlung von Magen-Darm-Erkrankungen (Collitis Ulcerosa, Morbus Krohn), zur Behandlung von Morbus Bechterew, zur Behandlung von chron. Schmerzen des Bewegungsapparats, zur Behandlung von Diabetes (Verbesserung der Blutzuckerwerte), zur Behandlung von Ödemen, zur Comedikation zu Opioiden oder anderen Analgetika.

Die Erfindung wird nachfolgend anhand von Beispielen verdeutlicht. Diese stellen jeweils nur eine von vielen möglichen Ausführungsformen der Erfindung dar.

### 1. Ausführungsbeispiel 1, Herstellung des Kohlensäure-Adduktes (KA)

### 1.1 Materialien:

- Amin (AM) : 68,8 bis 110,1 g Procainhydrochlorid (z.B. reinst, zur Verwendung als pharmazeutischer Wirkstoff; Ph. Eur. oder in einer Qualität, die für diesen Zweck geeignet ist)
- Base (BA): 22,2 bis 33,9 g Natriumhydrogencarbonat (z.B. reinst oder in einer Qualität, die für diesen Zweck geeignet ist),
- Lösungsmittel: 630 bis 900 ml Wasser (Aqua ad iniectiabilia)
- CO₂: mind. 12,0 g/l Kohlendioxid aus Druckgasstahlflaschen (CO₂ in geeigneter Qualität)
- Trockeneis zur Bereitung von Kältemischungen und zur Kühlung
- Methanol, techn. zur Bereitung von Kältemischungen

### 1.2 Schritt a)

In eine gereinigte Kunststoffdruckflasche wird Wasser (z.B. Aqua ad iniectiabilia) bis zur Markierung eingefüllt (ca. 800 bis 900 ml) und für mindestens 1h im Kühlschrank (3 bis 8 °C) oder mittels externer Kühlung auf 5 °C vorgekühlt.

Es wird eine mit Kohlendioxid gesättigte Kohlensäurelösung bereitet. Dafür wird CO₂ intervallweise unter Druck (1,6 bis 8 bar) in das vorgekühlte Wasser eingebracht. Das Zischen (entweichendes Gas über das Überdruckventil) zeigt Sättigung der Lösung mit CO₂ an. Die Sättigung wird über das Gewicht kontrolliert, bis 4,0 bis 6,0 g CO₂ (entsprechend 4,5 bis 7,5 g/l) gelöst sind. Die gesättigte Lösung weist einen pH-Wert von ≤ 4,3 bis 4,8 auf. Dieses Kohlensäure-haltige Wasser wird unmittelbar verschlossen sowie mindestens für 1h im Kühlschrank aufbewahrt.

### 1.3 Schritt b)

In einer zweiten Kunststoffdruckflasche wird 21,2 g Natriumhydrogencarbonat vorgelegt, mit 320 ml gekühltem CO₂₋haltigen Wasser versetzt und unter Schwenken aufgelöst.

### 1.4 Schritt c)

Zu dieser Lösung wird die äquivalente Menge an festem Procainhydrochlorid bei gleichbleibender Temperatur gegeben, wobei sich eine nahezu neutrale Lösung bildet, die nach Zugabe von weiteren 320 ml kalten kohlensäure-haltigen Wasser eine klare schwach saure Lösung ergibt. Die Lösung wird mit CO₂ angereichert. Die so bereitete Lösung wird für mindestens eine 1h im Kühlschrank gelagert.

Anschließend wird die Lösung nochmals mit CO₂ konditioniert, bis eine CO₂-Konzentration von 12g/l in der Lösung erreicht ist Mittels pH-Indikatorstäbchen wird der pH-Wert kontrolliert. Der pH-Wert beträgt ≤ 6,6.

### 1.5 Schritt d)

Rundkolben werden vorgekühlt. Zum Einfrieren wird die Reaktionslösung in einem vorgekühlten Messzylinder abgemessen, portionsweise in Rundkolben überführt und durch Eintauchen in eine Trockeneis/Methanol-Kältemischung (<-60°C) nach dem Shell-freezing-Verfahren innerhalb von 1,5-3,5 min pro Kolben eingefroren (-200 rpm). Der Eintauchwinkel des Kolbens am Rotationsverdampfer wird auf ca. 40° eingestellt.

### 1.6 Schritt e)

Die Kolben mit dem so eingefrorenen Gut werden mit einem Schliffstopfen verschlossen und einem Tiefkühlschrank bei -15 bis -20 °C 2 bis 4 Tage zwischengelagert.

### 1.7 Schritt f)

Die so temperierten Kolben werden mit Styroporbehältern ummantelt, die vorgekühlt sind und unverzüglich einzeln an eine evakuierte (0,060 ± 0,01 mbar, ca. -46 °C, Dichtheitsprobe) Gefriertrocknungsanlage, über einen flexiblen Gummikegel angeschlossen. Die Ventilhähne werden vorsichtig geöffnet und die einzelnen Kolben unter Vakuum gesetzt. Abschließend müssen alle Kolben evakuiert sein.

Zur Überwachung des Prozesses werden unten im Styropormantel Temperaturfühler platziert, die während der gesamten Trocknung den gesamten Temperaturverlauf aufzeichnen. Vor dem Start der Lyophilisation zeigen die Temperaturfühler Temperaturen von < -5 °C an.

Während der Lyophilisation beträgt der Druck 0,07 ± 0,02 mbar. Dieser Sublimationsdruck wird innerhalb von 4 h erreicht und während der gesamten Lyophilisationszeit beibehalten. Die Kühlkammer wird während der gesamten Trocknung auf 9 bis 15 °C temperiert. Der Endpunkt der Lyophilisation wird graphisch aus den Temperaturverlaufsaufzeichnungen ermittelt. Die Gesamttrocknungszeit betrug maximal 52 h. Das trockene Lyophilisat wird in ein Braunglasgefäß mit Twist-off-Deckel überführt, mit einem Trockenmittelbeutel versehen und im Kühlschrank bei 0 bis 15 °C gelagert.

### 2. Auswirkung der CO₂ Konzentration in der Lösung, die dem Schritt d) unterzogen wird und der Dauer der Zwischenlagerung in Schritt e) auf die Lagerstabilität und den Zersetzungspunkt des Kohlensäure-Addukts (KA)

Wie in Tabelle 1 zusammengefasst, wurde in den Ausführungsbeispielen 2.2 bis 2.8 gegenüber Ausführungsbeispiel 1 jeweils die Gesamtmenge an CO₂ in der Lösung, die dem Schritt d) unterzogen wird und/oder die Dauer der Zwischenlagerung in Schritt e) variiert.

**Tabelle 1: Gesamtmenge an CO₂ in der Lösung von Schritt d) und Lagerdauer in Schritt e) in den Ausführungsbeispielen 2 bis 8, sowie die resultierenden Zersetzungspunkte und Lagestabilitäten der jeweiligen Kohlensäure-Addukte (KA)**

| **Beispiel** | **Gesamtmenge CO₂ in der Lösung, von Schritt d) in g/l** | **Lagerdauer in Schritt e) in Tagen** | **Zersetzungspunkt des Kohlensäure-Addukts (KA) in °C** | **Lagerstabilität⁴ bei 3 bis 8 °C in Monaten** |
|---|---|---|---|---|
| 2.2² | ca.⁵ 5 | ca. 4 | 71-77 | 3 |
| .2.3² | ca. 4 | <1 | 69-76 | 9 |
| 2.4¹ | ca. 6,5 | ca. 3 | 70-82 | 12 |
| 2.5² | ca. 7,5 | ca. 5 | 69-81 | 3 |
| 2.6¹ | ca. 15,5 | ca. 4 | 85-90 | >25³ |
| 2.7¹ | ca. 14,5 | ca. 2,75 | 73-94 | >21³ |
| 2.8¹ | ca. 14,5 | <1 | 70-80 | 13 |

| | | | | |
|---|---|---|---|---|
| ¹Ausführungsbeispiel; ²Vergleichsbeispiel, nicht erfindungsgemäß ³Lagerversuch noch nicht abgeschlossen, Probe ist stabil ⁴Lagerung im geschlossenen Gefäß, mehrfache Öffnung nach Aufwärmen auf Raumtemperatur zur Probennahme ⁵der Ausdruck "ca." bezeichnet im Rahmen dieser Tabelle 1 einen Toleranzbereich von ±5%. | | | | |

Die Beispielspaarungen 2.2 und 2.6, 2.4 und 2.7 sowie auch die Beispiele 2.3 und 2.8, die annähernd die gleiche Lagerdauer in Schritt e) aufweisen, zeigen, dass es bei einer Verdopplung bis Verdreifachung der Gesamtmenge an CO₂ in der Lösung, die dem Schritt d) unterzogen wird, zu einer sehr signifikanten Erhöhung bis überproportionalen Erhöhung der Lagerstabilität kommt. So führt beispielsweise die Verdreifachung der Gesamtmenge an CO₂ in der Lösung, die dem Schritt d) unterzogen wird, in Beispiel 2.6 gegenüber Beispiel 2.2 mindestens zu einer Verachtfachung der Lagerstabilität. Es zeigt sich anhand von Beispiel 2.5, im Vergleich zu Beispiel 2.4 aber auch, dass eine Erhöhung der Lagerdauer in Schritt e) über 4 Tage, trotz höherer Gesamtmenge an CO₂ wieder zu einem Absinken der Lagerstabilität führt.

Eine Probe gilt nicht mehr als stabil, wenn mit IR-Spektroskopie die spezifischen Banden des freien Amins (AM), hier Procain, nachgewiesen werden können.

Die erhöhte Lagerstabilität korreliert auch mit einer Steigerung des Zersetzungspunktes. Vergleicht man die oben genannten 3 Beispielspaarungen miteinander, wird deutlich, dass die Dauer der Zwischenlagerung in Kombination mit der Gesamtmenge an CO₂ in der Lösung, die dem Schritt d) unterzogen wird, einen deutlichen Einfluss auf die Lagerstabilität hat. Insbesondere die Beispiele 2.6 und 2.7 zeigen eine besonders deutlich erhöhte Lagerstabilität und Erhöhung des Zersetzungspunktes.

### 3. Vergleichende DSC-Untersuchungen der Beispiele 2.8, 2.7 und 2.6

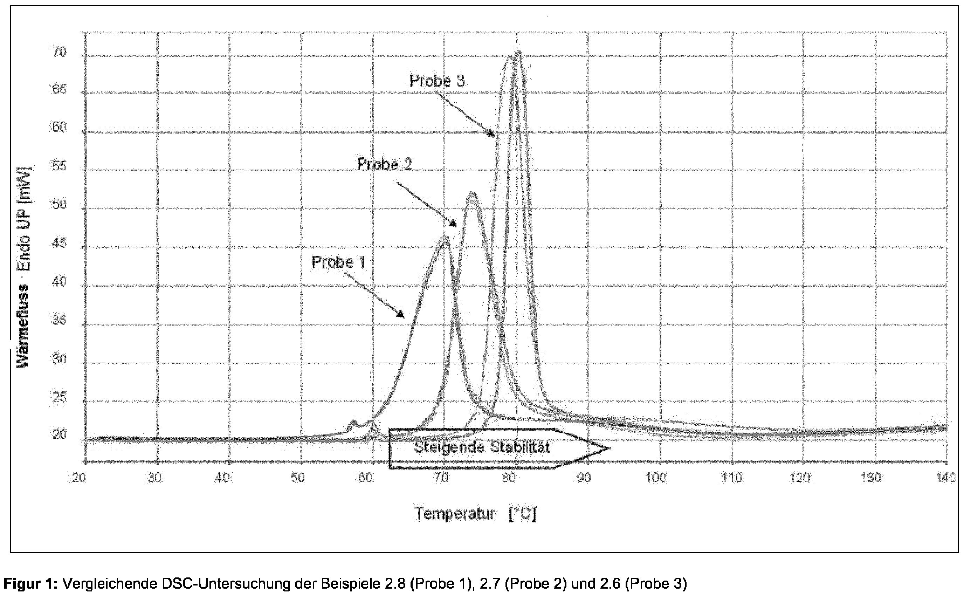

Mit den Kohlensäure-Addukten (KA) der Beispiele 2.8 (Probe 1 in Figur 1), 2.7 (Probe 2 in Figur 1), und 2.6 (Probe 3 in Figur 1) wurden vergleichende DSC-Untersuchungen (DSC = Differential Scanning Calorimetry) durchgeführt.

**Tabelle 2: Vergleichende DSC- Untersuchung**

| | Probe 1 | Probe 2 | Probe 3 |
|---|---|---|---|
| Peak-onset-Temp. Tₑ 1. Bestimmung [°C] | 61 | 69 | 75 |
| Peak-onset-Temp. Tₑ 2. Bestimmung [°C] | 61 | 69 | 77 |
| Peak-offset-Temp. T_{c} 1. Bestimmung [°C] | 74 | 80 | 83 |
| Peak-offset-Temp. T_{c} 2. Bestimmung [°C] | 74 | 81 | 83 |
| Peak-Maximum-Temp. Tₚ 1. Bestimmung [°C] | 70 | 74 | 79 |
| Peak-Maximum-Temp. Tₚ 2. Bestimmung [°C] | 70 | 74 | 80 |

Aus Figur 1 und Tabelle 1 ist von Probe 1 (Beispiel 2.8), über Probe 2 (Beispiel 2.7), zu Probe 3 (Beispiel 2.6) eine Verschiebung des Zersetzungspunktes zu höheren Temperaturen hin erkennbar. Somit steigt der Zersetzungspunkt mit Zunahme der Gesamtmenge an CO₂ in Schritt d), in Verbindung mit einer längeren Lagerdauer in Schritt e).

Je höher die Peak-onset-Temperatur und je schmaler der Peak umso höher ist das Ordnungsmaß innerhalb des Kohlensäure-Adduktes (KA) und umso stabiler ist das Produkt. Hier zeigen die gemessenen Daten, dass die Stabilität des Kohlensäure-Adduktes (KA) von Beispiel 2.8, über Beispiel 2.7 bis Beispiel 2.6 zunimmt.

### 4. Beispiele für Pharmazeutische Zubereitungen (PZ)

### 4.1 Kapseln und Tabletten

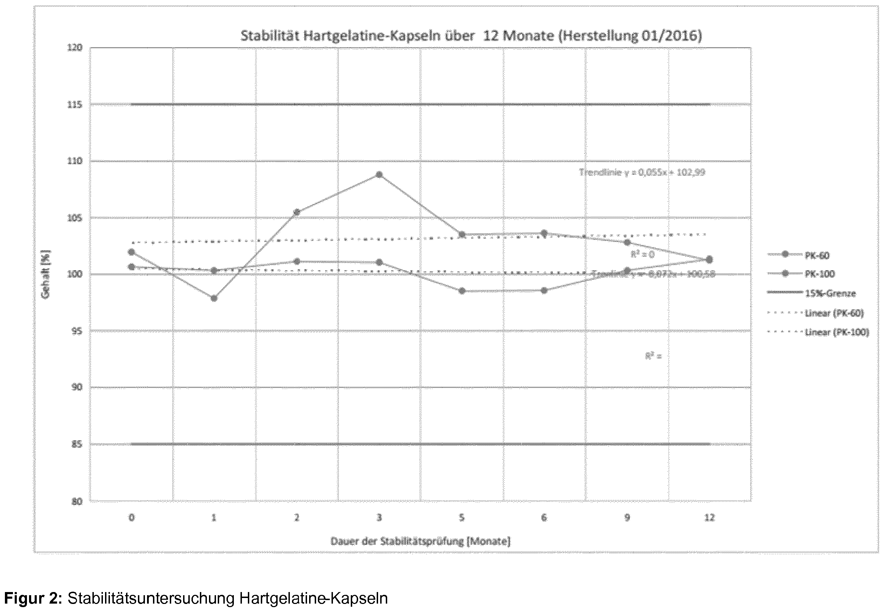

Nachfolgend wird beispielhaft die Zusammensetzung der erfindungsgemäßen pharmazeutischen Zubereitung (PZ) in der Ausführungsform als Kapsel oder Tablette für Procain als Amin (AM) beschrieben. Für die Herstellung der Kapseln können handelsübliche Steckkapseln in den handelsüblichen Größen (5 bis 000) verwendet werden, die mit dem Kohlensäure-Addukt (KA) haltigen Pulver, umfassend Procain als Amin (AM) (Verreibung des Wirkstoffs Kohlensäure-Addukt (KA), umfassend Procain als Amin (AM) ggf. mit Zusätzen, Füllstoffen, und Fließregulierungsmittel) befüllt werden. Das Kohlensäure-Addukt (KA) wurde gemäß Beispiel 1 hergestellt. Es hat sich gezeigt, dass Hartgelatine-Kapseln gegenüber Cellulose-Kapseln im Hinblick auf die Stabilität besser geeignet sind. So wiesen die befüllten Hartgelatinekapseln, beispielhaft für Hartgelatinekapseln mit 60 und 100 mg Wirkstoff gemäß Tabelle 3, auch nach 12 Monaten Lagerung im Kühlschrank keine Veränderungen auf und sind somit stabil (Figur 2). Die Stabilität wurde mittels IR-Spektroskopie untersucht. So wurde im Fall der Hartgelatine-Kapseln innerhalb des 12 Monatszeitraumes IR-spektroskopisch kein Procain detektiert, während für Cellulose-Kapseln bereits nach wenigen Tagen eine Procain-Bande im IR-Spektrum gemessen wurde. Die Gehaltsbestimmung erfolgte bei Raumtemperatur mit UV/VIS-Spektroskopie. Für Rezepturarzneimittel wird gemäß *Pharmacopoea Europaea* Punkt 2.9.6 bezogen auf den Gesamtgehalt inklusive Nebenprodukte ein Toleranzbereich von ±15 % vorgeschrieben. Die in Figur 2 dargestellten Schwankungen des Gehaltes sind somit Schwankungen die aus der Prozedur der Kapselherstellung resultieren.

Es werden die geltenden und als allgemein üblichen pharmazeutischen Regeln zur Herstellung von (Rezeptur)-Arzneimitteln angewendet (zum Beispiel Pharmacopoea Europaea, Deutscher Arzneimittel-Codex).

**Tabelle 3: Beispielzusammensetzung Kapseln mit NaHCO₃-Zusatz und Kohlensäure-Addukt (KA) als Wirkstoff hergestellt gemäß Ausführungsbeispiel 1.**

| Wirkstoffmenge [mg] | 30 | 60 | 100 | 120 |
|---|---|---|---|---|
| Zusatz, z.B. NaHCO₃ | 21 | 42 | 71 | 84 |
| Füllstoff, z.B. Maisstärke inkl. SiO₂ | 120 | 90 | 120 | 90 |
| Kapselgröße | 1 | 1 | 0 | 0 |

**Tabelle 4: Beispielzusammensetzung Tablette mit NaHCO₃-Zusatz und Kohlensäure-Addukt (KA) als Wirkstoff hergestellt gemäß Ausführungsbeispiel 1.**

| Wirkstoffmenge [mg] | 30 | 60 | 100 | 120 |
|---|---|---|---|---|
| Zusatz, z.B. NaHCO₃ | 21 | 42 | 71 | 84 |
| Füllstoff, z.B. Maisstärke inkl. SiO₂ | 2,5 | 5 | 8,5 | 10 |

### 4.2 Salben

Nachfolgend wird die Zusammensetzung der erfindungsgemäßen pharmazeutischen Zubereitung (PZ) in der Ausführungsform als Salbe beispielhaft für Procain als Amin (AM) im Kohlensäure-Addukt (KA) erläutert. Bei der Bereitung der Salbe werden die geltenden und allgemein üblichen pharmazeutischen Regeln zur Herstellung von (Rezeptur)-Arzneimitteln angewendet (zum Beispiel Pharmacopoea Europaea, Deutscher Azneimittel-Codex). Bei der Herstellung der Salbe werden größere Scherkräfte vermieden. Außerdem wird die Temperatur bei der Zubereitung auch lokal unter 60 °C gehalten. So wird das gemörserte Kohlensäure-Addukt (KA) umfassend Procain als Amin (AM) in einem Mörser oder einer Fantaschale, die mittels eines auf 40 bis 45 °C temperierten Wasserbades, temperiert werden, in die Salbengrundlage, beispielsweise Vaseline, eingebracht. Alternativ ist auch die Verwendung von elektrischen Mischsystemen möglich, wie sie im Rahmen des üblichen Apothekenbetriebes zum Einsatz kommen.

**Tabelle 5: Beispielzusammensetzung Salbe und Kohlensäure-Addukt (KA) als Wirkstoff hergestellt gemäß Ausführungsbeispiel 1.**

| | | | | | |
|---|---|---|---|---|---|
| Gehalt [%] | 0,5 | 1,0 | 1,25 | 2 | 4 |
| Wirkstoffmenge [mg] | 25 | 50 | 62,5 | 100 | 200 |
| Salbengrundlage [g] (z.B. Vaseline) | 5 | 5 | 5 | 5 | 5 |

### 4.3 Membrangängigkeit

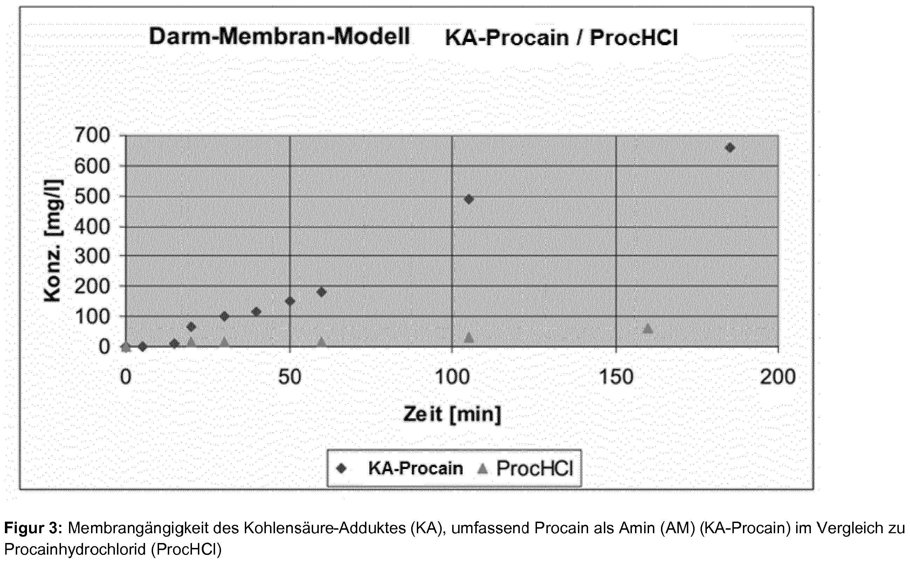

In einem Versuch mit Schweinedarm konnte die Membrangängigkeit des Kohlensäure-Addukts (KA) umfassend Procain als Amin (AM), gegenüber Procainhydrochlorid demonstriert werden. Die Ergebnisse sind in Figur 3 zusammengefasst.

Der Versuch wurde in Tyrode-Lösung am Schweinedarm (frisch vom Schlachthof geholt) durchgeführt. Dazu wurde der Darm mit Tyrodelösung gespült und auf einen Trichter gespannt. Die Darminnenseite tauchte in ein Gefäß ein, in dem sich Tyrodelösung mit Kohlensäure-Addukt (KA) mit Procain als Amin, hergestellt wie in Ausführungsbeispiel 1 beschrieben, bzw. Procainhydrochlorid (äquimolar) befanden. Auf die Darmaußenseite wurde ebenfalls Tyrodelösung ohne Substanz gegeben, um den Darm "frisch" zu halten und ihn mit Nährstoffen zu versorgen. Aus der oberen Lösung (Darmaußenseite) wurden die Proben entnommen und UV/VIS-spektroskopisch untersucht. Der gesamte Versuch wurde bei 35-38°C im Wasserbad temperiert durchgeführt.

### 4.4 Parenterale Lösungen

Für die Bereitung einer parenteralen Lösung die das Kohlensäure-Addukt (KA) umfassend Procain als Amin (AM) enthält, wird in einem geeigneten Gefäß mit einem Rührfisch oder Ähnlichem die erforderliche Menge Wasser (Aqua ad iniectabilia) auf ca. 5 ±3 °C gekühlt und bei dieser Temperatur belassen. Das Wasser wird mit gasförmigen Kohlendioxid in der benötigten Qualität auf ca. 3,2 g/l angereichert. In diesem CO₂-haltigen Wasser wird die entsprechende Menge Kohlensäure-Addukt (KA) umfassend Procain als Amin (AM) und Natriumchlorid für einen isotonischen Gehalt gelöst.

Alternativ wird auf ca. 5 ± 3 °C temperiertes Wasser unter Druck in einem geschlossenen System mit CO₂ so angereichert, dass ein deutlicher Überschuss vorhanden ist (4,5 bis 7,5 g/l). Zu diesem kohlensäurehaltigen Wasser werden ebenfalls die entsprechenden Mengen Kohlensäure-Addukt (KA) umfassend Procain als Amin (AM) und Natriumchlorid gegeben.

Diese kalte mit dem Kohlensäure-Addukt (KA) umfassend Procain als Amin (AM) und Natriumchlorid versehene Lösung wird unter geeigneten räumlichen Bedingungen sterilfiltriert und in entsprechende Vials abgefüllt. Es werden die geltenden und allgemein üblichen pharmazeutischen Regeln zur Herstellung von (Rezeptur)-Arzneimitteln angewendet (zum Beispiel Pharmacopoea Europaea).

**Tabelle 6: Beispielzusammensetzungen für parenterale Zubereitungen mit NaCl-Zusatz und Kohlensäure-Addukt (KA) als Wirkstoff hergestellt gemäß Ausführungsbeispiel 1.**

| | Infusion | | | Injektion | | |
|---|---|---|---|---|---|---|
| Gehalt [%] | 0,1 | 0,2 | 0,3 | 1 | 2 | 3 |
| Wirkstoffmenge [mg] | 50 | 100 | 150 | 50 | 100 | 150 |
| Zusatz (NaCl) [mg] | 440 | 430 | 425 | 37 | 29 | 25 |
| Volumen gesamt [ml] | 50 | 50 | 50 | 5 | 5 | 5 |

### 4.4.1 Stabilität parenteraler Lösungen

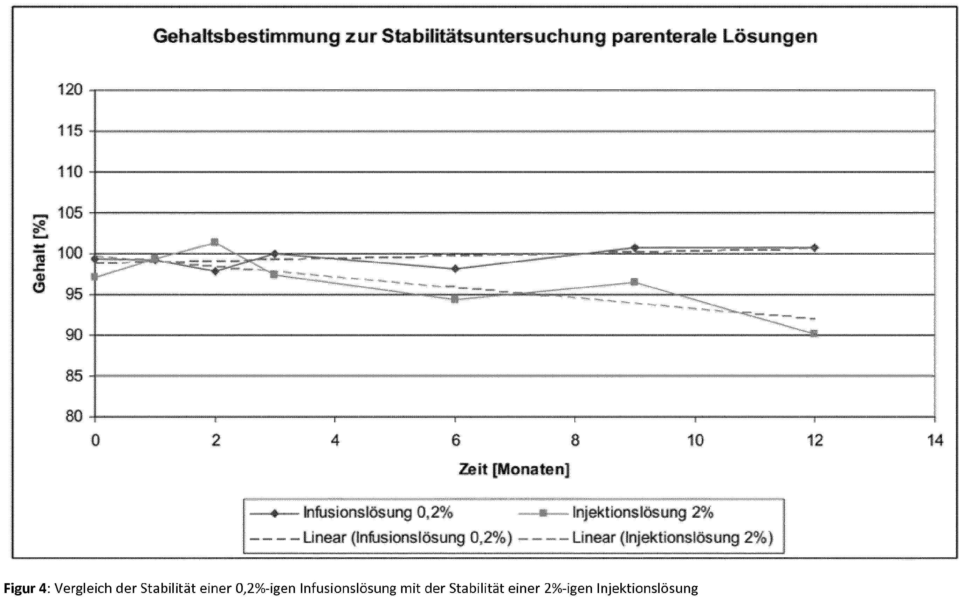

In einem Echtzeitversuch wurde die Stabilität einer 0,2%-igen Infusionslösung mit isotonischem NaCI-Gehalt mit einer 2%-igen Injektionslösung ohne weitere Zusätze verglichen. Wie in Figur 4 dargestellt, wird jeweils die Toleranzgrenze von ±15% für den Gesamtgehalt über den gesamten Zeitraum von 12 Monaten nicht tangiert. Anders als in der 0,2%-igen Infusionslösung, in der das Zersetzungsprodukt des Procains, p-Aminobenzoesäure (pABA), über die gesamten 12 Monate nicht nachgewiesen werden kann, wird für die 2%-ige Infusionslösung ab 6 Monaten, bis zu 12 Monaten innerhalb der Toleranzgrenze, die zunehmende Bildung von Aminobenzoesäure (pABA) beobachtet.

Zur Ermittlung der entsprechenden IR-Daten wurden die Lösungen ohne weiteren CO₂-Zusatz vor dem Einfrieren, um das Ergebnis nicht zu verfälschen, eingefroren und lyophilisiert, wie in Ausführungsbeispiel 1 beschrieben. Für den so erhaltenen Feststoff wurden die entsprechenden IR-Spektren gemessen, die während des betrachteten Zeitraumes keine Procainbanden aufwiesen. Der Gesamtgehalt wurde jeweils zweimal, aus der Lösung und dem Lyophilisat UV/VIS-spektroskopisch bestimmt.

### 4.4.2 Einfluss des Natriumchlorids und des CO₂ auf die Stabilität parenteraler Lösungen

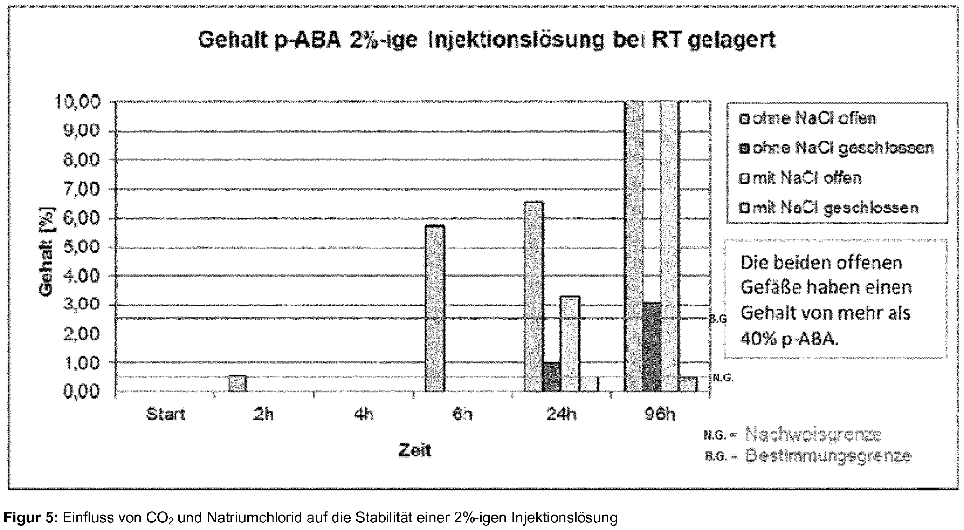

Der Einfluss des Natriumchlorids und des gelösten CO₂ auf die Lagerstabilität von Injektionslösungen wurde untersucht. Dazu wurden 2-%-ige Injektionslösungen, wie in 4.4. beispielhaft beschrieben, bei Raumtemperatur, mit und ohne Natriumchloridzusatz, jeweils in offenen und geschlossenen Gefäßen gelagert. In Abständen wurden Proben entnommen und der Gehalt an p-Aminobenzoesäure, einem Zersetzungsprodukt des Procains, bestimmt. Wie aus Figur 5 hervorgeht, zeigen die Versuche deutlich, dass sich die isotonische Kochsalzlösung günstig auf die Stabilität der Lösungen auswirkt. So wurde in den Proben der geschlossenen Vials mit isotonischem Natriumchloridgehalt auch nach 4 Tagen bei Raumtemperatur keine nennenswerte Menge an p-Aminobenzoesäure gemessen. Ohne den Natriumchloridzusatz wurde deutlich p-Aminosäure detektiert

Bei den offenen Proben wurde dieser Effekt ebenfalls aber bereits nach viel kürzerer Zeit festgestellt. Alle Proben in offenen Gefäßen sind nach 4 Tagen deutlich zersetzt. Das deutet zusammen mit den Befunden der geschlossenen Vials darauf hin, dass beide Zusätze, sowohl CO₂ als auch das NaCl (isotonisch), sich positiv auf die Stabilität der der Injektionslösungen auswirken. Der Einfluss des CO₂ ist deutlich ausgeprägter als der des isotonischen NaCI-Gehalts.

## Patentansprüche

1. Kohlensäure-Addukt (KA) umfassend Kohlensäure, mindestens ein Amin (AM) und optional mindestens ein Salz (S),
herstellbar nach einem Verfahren umfassend die Schritte
a) Bereitstellung einer Lösung (A), die mindestens ein Lösungsmittel und in dem mindestens einem Lösungsmittel gelöstes CO₂ umfasst,
optional b) Auflösen einer Base (BA), die nicht dem Amin (AM) entspricht, in der Lösung (A) unter Erhalt der Lösung (A1),
c) Lösen des mindestens einen Amins (AM) in der Lösung (A) oder (A1), unter Erhalt der Lösung (B),
d) Einfrieren der nach Abschluss des Schrittes c) erhaltenen Lösung,
e) Lagerung der in Schritt d) eingefrorenen Lösung bei -100 bis 0 °C für nicht länger als 4 Tage,
wobei der Gehalt an CO₂ in der Lösung, die dem Schritt d) unterzogen wird, mindestens 6 g/l, vorzugsweise mindestens 10 g/l, mehr bevorzugt mindestens 12 g/l, noch mehr bevorzugt mindestens 14g/l und ganz besonders bevorzugt mindestens 15 g/l beträgt und das Amin (AM) auch in Form eines Salzes eingesetzt werden kann.

2. Das Kohlensäure-Addukt (KA) gemäß Anspruch 1, wobei Schritt a) mindestens einen der folgenden Teilschritte umfasst:
a1) Kühlen des Lösungsmittels, vorzugsweise Wasser, auf 3 bis 8 °C, vorzugsweise 5 °C und/oder
a2) Einbringen von CO₂ in das Lösungsmittel, vorzugsweise bis zu einer Sättigungskonzentration von 3 bis 10 g/l, mehr bevorzugt bis zu einer Sättigungskonzentration 4,5 bis 7,5 g/l, vorzugsweise beträgt der pH-Wert der Lösung nach der Sättigung mit CO₂ ≤ 3,0 bis 6,0, noch mehr bevorzugt ≤ 4,3 bis 4,8 und/oder
a3) Lagerung der Lösung (A) bei 1 bis 10°C, vorzugsweise für mindestens 30 min, mehr bevorzugt für mindestens 50 min, noch mehr bevorzugt für mindestens 60 min; bis höchstens 5d (120h); vorzugsweise erfolgt die Lagerung bei 3 bis 8 °C vorzugsweise für mindestens 30 min, mehr bevorzugt für mindestens 50 min, noch mehr bevorzugt für mindestens 60 min; bis höchstens 5d (120h);
vorzugsweise umfasst Schritt a) alle Teilschritte a1), a2) und a3), vorzugsweise werden die Teilschritte a1), a2) und a3) in der Reihenfolge a2) folgt auf a1) und a3) folgt auf a2) durchgeführt und/oder
wobei die Base (BA) in Schritt b) ein Hydrogencarbonat oder ein Carbonat, mehr bevorzugt ein Hydrogencarbonat, noch mehr bevorzugt Natriumhydrogencarbonat ist.

3. Das Kohlensäure-Addukt (KA) gemäß einem der Ansprüche 1 bis 2, wobei Schritt c) mindestens einen der folgenden Teilschritte umfasst:
c1) Auflösen des mindestens einen Amins (AM) in Lösung (A) oder (A1) unter Erhalt der Lösung (B) und/oder
c2) Zugabe von Lösung (A) zur Lösung (B) unter Erhalt der Lösung (B1) und/oder c3) Anreicherung der Lösung (B) oder (B1) mit CO₂ und/oder
c4) Lagerung der Lösung (B) oder (B1) bei 1 bis 10°C, vorzugsweise 3 bis 8 °C, für mindestens 1 h vorzugsweise 24 h bis 120 h, noch mehr bevorzugt 24 bis 72 h, und/oder
c5) Anreicherung der Lösung (B) oder (B1) mit CO₂ auf eine Konzentration von mindestens 6 g/l, vorzugsweise mindestens 10 g/l, mehr bevorzugt mindestens 12 g/l, noch mehr bevorzugt mindestens 14 g/l und ganz besonders bevorzugt mindestens 15 g/l;
wobei optional
i) die Konzentration des Amins (AM) in Lösung (B) oder bei Ausführung von Teilschritt c2) in Lösung (B1) 0,01 bis 0,25 g/ml, vorzugsweise 0,03 bis 0,20 g/ml, mehr bevorzugt 0,08 bis 0,15 g/ml beträgt und/oder
ii) der pH-Wert der Lösung (B) oder (B1) nach der Durchführung von Schritt c5) ≤ 7,0 beträgt und/oder
iii) das Verhältnis des Amins (AM) zur Base (BA), bei Durchführung des Schrittes b), in Lösung (B) 2:1 bis 5:1, mehr bevorzugt 3:1 bis 4:1, noch mehr bevorzugt 3,23:1 bis 3,26:1 [g/g] beträgt und/oder
iv) das molare Verhältnis des Amins (AM) zu den Basenequivalenten der Base (BA), bei Durchführung von Schritt b), in Lösung (B) 0,8:1 bis 1,5:1, vorzugsweise 1,2:1, mehr bevorzugt 1:1 beträgt und/oder
v) in Schritt c1) das mindestens eine Amin (AM), das mindestens eine Amin (AM) als Säureadditionssalz, vorzugsweise als Hydrohalogenid, Hydrogensulfat, Hydrogensulfit, Hydrogenphosphat, Hydromesylat, Hydrotosylat, Hydroacetat, Hydroformiat, Hydropropanoat, Hydromalonat, Hydrosuccinat, Hydrofumarat, Hydroxalat, Hydrotartrat, Hydrocitrat, Hydromaleat, mehr bevorzugt als Hydrochlorid oder Hydrobromid umfasst.
vorzugsweise umfasst Schritt c) alle Teilschritte c1), c2), c3), c4)und c5);
vorzugsweise werden die Teilschritte c1), c2), c3), c4) und c5) in der Reihenfolge c2) folgt auf c1), c3) folgt auf c2), c4) folgt auf c3), c5) folgt auf c4) durchgeführt.

4. Das Kohlensäure-Addukt (KA) gemäß einem der Ansprüche 1 bis 3, wobei in Schritt d):
i) die Lösung (B) oder (B1) bei -100 °C bis -20 °C, vorzugsweise bei -90 °C bis -30 °C, noch mehr bevorzugt bei -80 bis -40 °C und ganz besonders bevorzugt bei -70 bis -50 °C eingefroren wird und/oder
ii) die Lösung (B) oder (B1) innerhalb von 0,3 bis 60 Minuten, vorzugsweise innerhalb von 1 bis 30 Minuten, mehr bevorzugt innerhalb von 1,1 bis 10 Minuten, noch mehr bevorzugt innerhalb von 1,5 bis 5 Minuten eingefroren wird und/oder
iii) das Gefäß in dem sich die Lösung (B) oder (B1) während des Einfriervorgangs befindet, vorzugsweise im Kühlmedium, mit 10 bis 1000 rpm, vorzugsweise mit 50 bis 600 rpm, mehr bevorzugt mit 100 bis 400 rpm und noch mehr bevorzugt mit 200 bis 300 rpm rotiert wird und/oder
iv) die Lösung (B) oder (B1) mit einer Kühlrate von 10 bis 100 K/min, vorzugsweise mit 20 bis 80 K/min, mehr bevorzugt mit 30 bis 70 K/min und besonders bevorzugt mit 40 bis 60 K/min eingefroren wird und/oder
wobei in Schritt e):
i) die eingefrorene Lösung (B) oder (B1) für 1,5 bis 4 Tage, vorzugsweise für 2,5 bis 4 Tage gelagert wird und/oder
ii) die eingefrorene Lösung (B) oder (B1) vorzugsweise bei -50 bis 0 °C, mehr bevorzugt bei -30 bis -5 °C, noch mehr bevorzugt bei -25 bis -10 °C, besonders bevorzugt bei -20 bis - 15 °C gelagert wird.

5. Das Kohlensäure-Addukt (KA) gemäß einem der Ansprüche 1 bis 4, wobei das Verfahren einen weiteren Schritt f) umfasst, der nach Schritt e) durchgeführt wird,
f) Trocknung der in Schritt e) gelagerten Lösung unter Erhalt von getrocknetem Kohlensäure-Addukt (KA),
wobei in Schritt f) optional
i) das Wasser aus der Lösung (B) oder (B1) bis zu einem Restgehalt von < 0,8 Gew.-%, vorzugsweise < 0,1 Gew.-% bezogen auf das Gesamtgewicht des Trocknungsproduktes (C) entfernt wird und/oder
ii) nicht im Kohlensäure-Addukt (KA) gebundenes CO₂ aus der Lösung (B) oder (B1) bis zu einem Restgehalt von < 0,8 Gew.-%, vorzugsweise < 0,1 Gew.-% bezogen auf das Gesamtgewicht des Trocknungsproduktes (C) entfernt wird und/oder
iii) die Trocknung mittels Lyophilisation durchgeführt wird und/oder
iv) während des Trocknens der Druck 0,01 bis 30 mbar, vorzugsweise 0,02 bis 20 mbar, mehr bevorzugt 0,03 bis 10 mbar, noch mehr bevorzugt 0,03 bis 0,5 mbar und ganz besonders bevorzugt 0,05 bis 0,1 mbar beträgt und vorzugsweise während des gesamten Trocknungsvorganges beibehalten wird und/oder
v) der Druck während des Trocknens gemäß iv) innerhalb von 10 h, vorzugsweise innerhalb von 7 h, mehr bevorzugt innerhalb von 5 h und besonders bevorzugt innerhalb von 4 h ab Evakuierungsbeginn erreicht wird und/oder
vi) die Temperatur während der gesamten Trocknung in Schritt f) 0 bis 20 °C, vorzugsweise 4 bis 18 °C, mehr bevorzugt 8 bis 16 °C beträgt und/oder
vii) die Gesamttrocknungszeit 10 bis 60 h, vorzugsweise 30 bis 55 h, mehr bevorzugt 41 bis 52 h beträgt.

6. Das Kohlensäure Addukt (KA) gemäß einem der Ansprüche 1 bis 5, wobei
a) mindestens ein Amin (AM) gemäß einer der nachstehenden Formeln (I) oder (II) eingesetzt wird, 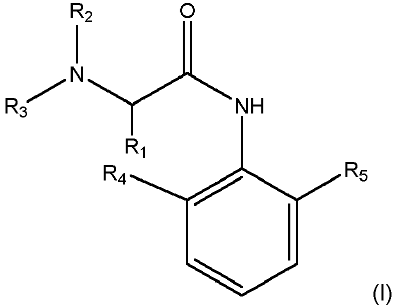 wobei in Formel (I)
R₁ ist H, C₁₋₁₀Alkyl, C₂₋₁₀Alkenyl, Aryl oder Heteroaryl, vorzugsweise H oder C₁₋₁₀Alkyl, mehr bevorzugt H oder C₁₋₄Alkyl;
R₂ ist H, C₁₋₁₀Alkyl, C₂₋₁₀Alkenyl, Aryl oder Heteroaryl, vorzugsweise H oder C₁₋₁₀Alkyl, mehr bevorzugt H oder C₁₋₄Alkyl;
R₃ ist H, C₁₋₁₀Alkyl, C₂₋₁₀Alkenyl, Aryl oder Heteroaryl, vorzugsweise H oder C₁₋₁₀Alkyl, mehr bevorzugt H oder C₁₋₄Alkyl;
wobei optional R₁ und R₃ miteinander verbunden sein können und zusammen mit dem Stickstoffatom an das R₃ gebunden ist und dem Kohlenstoffatom an das R₁ gebunden ist, einen gesättigten oder ungesättigten, vorzugsweise einen gesättigten Ring bilden können, vorzugsweise ist der Ring 4, 5 oder 6 gliedrig, mehr bevorzugt 5 oder 6 gliedrig,
R₄ ist H, Halogen, C₁₋₁₀ Alkyl, C₂₋₁₀ Alkenyl, Aryl oder Heteroaryl, vorzugsweise H oder Methyl;
R₅ ist H, Halogen, C₁₋₁₀Alkyl, C₂₋₁₀Alkenyl, Aryl oder Heteroaryl, vorzugsweise H, Methyl oder Halogen; 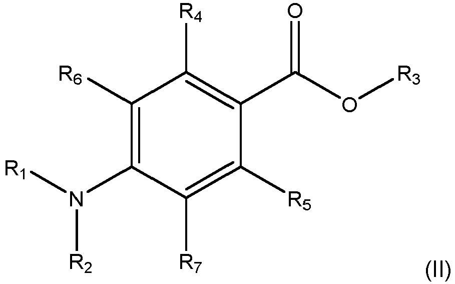 wobei in Formel (II)
R₁ ist H, C₁₋₁₀Alkyl, C₂₋₁₀Alkenyl, Aryl oder Heteroaryl, vorzugsweise H oder C₁₋₁₀Alkyl, mehr bevorzugt H;
R₂ ist H, C₁₋₁₀Alkyl, C₂₋₁₀Alkenyl, Aryl oder Heteroaryl, vorzugsweise H oder C₁₋₁₀ Alkyl, mehr bevorzugt H:
R₃ ist H, C₁₋₁₀ Alkyl, C₂₋₁₀ Alkenyl, Aryl, Heteroaryl oder -(CH₂)ₙNR₈R₉, vorzugsweise - (CH₂)ₙNR₈R₉,
n ist 1 bis 5, vorzugsweise 1 bis 3, mehr bevorzugt 1 bis 2,
R₈ ist C₁₋₁₀Alkyl, vorzugsweise C₁₋₂Alkyl,
R₉ ist C₁₋₁₀Alkyl, vorzugsweise C₁₋₂Alkyl;
R₄ ist H, Halogen, C₁₋₁₀Alkyl, C₂₋₁₀Alkenyl, Aryl, Heteroaryl oder -O-C₁₋₁₀Alkyl, vorzugsweise H, Halogen, C₁₋₁₀Alkyl oder -O-C₁₋₁₀Alkyl, mehr bevorzugt H oder Halogen;
R₅ ist H, Halogen, C₁₋₁₀Alkyl, C₂₋₁₀Alkenyl, Aryl, Heteroaryl oder -O-C₁₋₁₀Alkyl, vorzugsweise H, Halogen, C₁₋₁₀Alkyl oder -O-C₁₋₁₀Alkyl, mehr bevorzugt H oder Halogen;
R₆ ist H, Halogen, C₁₋₁₀Alkyl, C₂₋₁₀Alkenyl, Aryl, Heteroaryl oder -O-C₁₋₁₀Alkyl, vorzugsweise H, Halogen, C₁₋₁₀Alkyl oder-O-C₁₋₁₀Alkyl, mehr bevorzugt H oder -O-C₁₋₁₀Alkyl;
R₇ ist H, Halogen, C₁₋₁₀Alkyl, C₂₋₁₀Alkenyl, Aryl, Heteroaryl oder -O-C₁₋₁₀Alkyl, vorzugsweise H, Halogen, C₁₋₁₀Alkyl oder-O-C₁₋₁₀Alkyl, mehr bevorzugt H oder -O-C₁₋₁₀Alkyl;
wobei das mindestens eine Amin gemäß Formel (I) und (II) optional auch in Form eines Salzes eingesetzt werden kann oder
b) mindestens ein Amin (AM) eingesetzt wird, ausgewählt aus der Gruppe 4-Aminobenzoesäure-2-(*N,N*-diethylamino-)ethylester (Procain), 4-Aminobenzoesäureethylester (Benzocain), 2-(Diethylamino)ethyl-4-amino-2-chlorbenzoat (Chlorprocain), 4-Amino-3-butoxybenzoesäure-2-diethylaminoethylester (Oxybuprocain), (2-(Dimethylamino)ethyl)-4-(butylamino)benzoat (Tetracain), *N*-[3-(4-Phenoxymethylphenyl)propyl]morpholin (Fomocain), 2-Diethylamino-*N*-(2,6-dimethylphenyl)acetamid (Lidocain), (*RS*)-*N*-(2,6-Dimethylphenyl)-1-methylpiperidin-2-carboxamid (Mepivacain), (*RS*)-*N*-(2-Methylphenyl)-2-(propylamino)-propanamid (Prilocain), (*RS*)-4-Methyl-3-[2-(propylamino)-propanamido]thiophen-2-carbonsäuremethylester (Articain), (±)-1-Butyl-*N*-(2,6-dimethylphenyl)-2-piperidincarboxamid (Bupivacain), (*S*)-1-Propyl-2',6'-dimethyl-2-piperidylcarboxyanilid (Ropivacain), 2-(Ethylpropylamino)-2',6'-butyroxylidid (Etidocain), 1-(4-Butoxyphenyl)-3-piperidin-1-ylpropan-1-on (Dyclonin), vorzugsweise 4-Aminobenzoesäure-2-(*N*,*N*-diethylamino-)ethylester (Procain) und 2-Diethylamino-*N*-(2,6-dimethylphenyl)acetamid (Lidocain) (2-(Dimethylamino)ethyl)-4-(butylamino)benzoat (Tetracain) und/oder der Salze dieser Verbindungen.

7. Verfahren zur Herstellung des Kohlensäure-Addukts (KA) gemäß einem der Ansprüche 1 bis 6, umfassend die Schritte gemäß einem der Ansprüche 1 bis 6.

8. Kohlensäure-Addukt (KA), umfassend mindestens ein Amin (AM), Kohlensäure und optional mindestens ein Salz (S), herstellbar gemäß dem Verfahren nach Anspruch 7, wobei
i) das Kohlensäure-Addukt (KA),
Procain, Kohlensäure und mindestens ein Salz (S) umfasst und der Zersetzungspunkt 65 bis 95 °C, vorzugsweise 70 bis 90°C, mehr bevorzugt 85 bis 90 °C beträgt oder
Lidocain, Kohlensäure und mindestens ein Salz (S) umfasst und der Zersetzungspunkt 20 bis 45 °C, vorzugsweise 25 bis 45 °C, mehr bevorzugt 30 bis 45 °C beträgt und /oder
ii) das Kohlensäure-Addukt (KA) bei Lagerung bei einer Temperatur von 2 bis 10 °C für mindestens 12 Monate, vorzugsweise für mindestens 13 Monate, mehr bevorzugt für mindestens 20 Monate, noch mehr bevorzugt für mindestens 23 Monate, besonders bevorzugt für mindestens 27 Monate stabil bleibt.

9. Eine pharmazeutische Zubereitung (PZ) umfassend das Kohlensäure-Addukt (KA) nach einem der Ansprüche 1 bis 6 und 8 wobei optional
i) bei der Zubereitung der pharmazeutischen Zubereitung (PZ)
a) die Temperatur weniger als 60 °C, vorzugsweise weniger als 50 °C, mehr bevorzugt 0° C bis 50 °C beträgt und/oder
bei Anwendung von Dispergierern und/oder Salbenbereitern eine Drehzahl von < 2000 rpm verwendet wird;
wobei die pharmazeutische Zubereitung (PZ) optional
A) eine halbfeste Zubereitung ist und kutan appliziert wird und die pharmazeutische Zubereitung (PZ) optional
i) in Salbenform, als Creme, als Gel, als Paste, Umschlagpaste oder wirkstoffhaltiges Pflaster appliziert wird und/oder
ii) mindestens ein Hilfsstoff (H1) umfasst, ausgewählt aus Paraffine, insbesondere dick- und dünnflüssige Paraffine, Wollwachs, Wollwachsalkohole, hydrophobes Basisgel, pflanzliche Öle, tierische Fette, synthetische Glyceride, flüssige Polyalkylsiloxane, Wachse, Vaseline, Stärke, insbesondere Maisstärke vorzugsweise Vaseline und/oder
iii)
a) 0,1 bis 40 Gew.-%, vorzugsweise 0,4 bis 10 Gew.-% des Kohlensäure-Addukts (KA) und
b) 60 bis 99,9 Gew. -%, vorzugsweise 80 bis 96 Gew.-% des Hilfsstoffs (H1) bezogen auf die Gesamtmenge der pharmazeutischen Zubereitung (PZ) umfasst oder
B) die pharmazeutische Zubereitung (PZ) oral appliziert wird und die pharmazeutische Zusammensetzung (PZ) optional
i) in Kapseln, vorzugsweise in Hartgelatine- oder Cellulose- Kapseln, mehr bevorzugt in Hartgelatine Kapseln verabreicht wird und/oder
ii) in Tablettenform verabreicht wird und/oder
iii) mindestens ein Hilfsstoff (H) umfasst, vorzugsweise ausgewählt aus Stärke, insbesondere. Maisstärke und/oder Reisstärke, Dextran, Celluloseester und SiO₂ und/oder
iv) mindestens eine Base (BA1) umfasst, die nicht dem Amin (AM) entspricht und identisch oder verschieden von der Base (BA) ist, vorzugsweise ist die Base (BA1) ausgewählt aus NaHCO₃ oder KHCO₃, mehr bevorzugt NaHCO₃ und/oder;
v)
a) 1 bis 99 Gew.-%, vorzugsweise 15 bis 95 Gew.-% des Kohlensäure-Addukts (KA),
b) 0 bis 60 Gew.-%, vorzugsweise 3 bis 50 Gew.-% der Base (BA1) und
c) 1 bis 90 Gew.-%, vorzugsweise 2 bis 75 Gew. -% des Hilfsstoffes (H)
bezogen auf das Gesamtgewicht der pharmazeutischen Zubereitung umfasst oder
C) die pharmazeutische Zubereitung (PZ) parenteral, nasal und/oder inhalativ appliziert wird und die pharmazeutische Zubereitung (PZ) optional
I) ein Hilfsstoff (H2) umfasst, vorzugsweise ausgewählt aus einem Alkalihalogenid oder Erdalkalihalogenid, mehr bevorzugt aus NaCl und MgCl₂, noch mehr bevorzugt aus NaCl und/oder
II)
a) 0,05 bis 100 mg/ml, vorzugsweise 0,08 bis 50 mg/ml, des Kohlensäure-Addukts (KA), und
b) 0 bis 20 mg/ml, vorzugsweise 3 bis 10 mg/ml des Hilfsstoffs (H2)
bezogen jeweils auf das Gesamtvolumen der pharmazeutischen Zubereitung (PZ) umfasst.

10. Verfahren zur Herstellung der pharmazeutischen Zubereitung (PZ) gemäß Anspruch 9 B), umfassend die Schritte:
a) Bereitstellung einer Mischung umfassend das Kohlensäure-Addukt (KA) und optional mindestens eine Base (BA1) und/oder mindestens einen Hilfsstoff (H),
b) Zerreiben der Mischung zu Pulver,
c) Verarbeitung des Pulvers in eine Darreichungsform zur oralen Applikation, wobei optional
i) der mindestens eine Hilfsstoff (H) ausgewählt ist aus Stärke, insbesondere Maisstärke und/oder Reisstärke, Dextran, Celluloseester und SiO₂ und/oder
ii) die Base (BA1) ausgewählt ist aus NaHCO₃ oder KHCO₃, mehr bevorzugt NaHCO₃ und/oder
iii) die Darreichungsform zur oralen Applikation eine Tablette ist und/oder
iv) die Darreichungsform zur oralen Applikation eine Kapsel, vorzugsweise eine Hartgelatine- oder Cellulose- Kapsel, mehr bevorzugt eine Hartgelatine-Kapsel ist und/oder
v) vom Kohlensäure-Addukt (KA) 1 bis 99 Gew.-%, vorzugsweise 15 bis 95 Gew.-% bezogen auf das Gesamtgewicht der pharmazeutischen Zubereitung (PZ) eingesetzt werden und/oder
vi) von der Base (BA1) 0 bis 60 Gew.-%, vorzugsweise 3 bis 50 Gew.-% bezogen auf das Gesamtgewicht der pharmazeutischen Zubereitung (PZ) eingesetzt werden und/oder
vii) von dem Hilfsstoff (H) 1 bis 90 Gew.-%, vorzugsweise 2 bis 75 Gew. -% bezogen auf das Gesamtgewicht der pharmazeutischen Zubereitung (PZ) eingesetzt werden.

11. Verfahren zur Herstellung der pharmazeutischen Zubereitung (PZ) gemäß Anspruch 9 A), umfassend die Schritte
a) Zerreiben des Kohlenstoff-Adduktes (KA) zu Pulver,
b) Vermischen des Kohlenstoff-Adduktes (KA)-Pulvers aus Schritt a) mit mindestens einem Hilfsstoff (H1), wobei optional
i) der mindestens eine Hilfsstoff (H1) ausgewählt ist aus Paraffine, insbesondere dick- und dünnflüssige Paraffine, Wollwachs, Wollwachsalkohole, hydrophobes Basisgel, pflanzliche Öle, tierische Fette, synthetische Glyceride, flüssige Polyalkylsiloxane, Wachse, Vaseline, Stärke, insbesondere Maisstärke vorzugsweise Vaseline und/oder
ii) die Temperatur in Schritt a) und b) weniger als 60 °C, vorzugsweise weniger als 50 °C, mehr bevorzugt 5 bis 50 °C beträgt und/oder
iii) bei Anwendung von Dispergierern und/oder Salbenbereitern eine Drehzahl von < 2000 rpm verwendet wird und/oder
iv) 0,1 bis 40 Gew.-%, vorzugsweise 0,4 bis 10 Gew.-% des Kohlensäure-Addukts (KA) bezogen auf die Gesamtmenge der pharmazeutischen Zubereitung eingesetzt werden und/oder
v) 60 bis 99,9 Gew. -%, vorzugsweise 80 bis 96 Gew.-% des Hilfsstoffs (H1) bezogen auf die Gesamtmenge der pharmazeutischen Zubereitung (PZ) eingesetzt werden.

12. Verfahren zur Herstellung der pharmazeutischen Zubereitung gemäß Anspruch 9 C) umfassend die Schritte:
a) Bereitstellung einer Lösung (A2),
b) Auflösen des Kohlensäure-Addukts (KA) in der Lösung (A2),
wobei optional
i) die Lösung (A2) vorzugsweise durch Einbringen von CO₂ in ein Lösungsmittel erhalten wird und/oder
ii) das Lösungsmittel der Lösung (A2) Wasser ist und/oder
iii) CO₂ zur Herstellung der Lösung (A2) bei einer Temperatur von 0 bis 8 °C, mehr bevorzugt bei 0 bis 5 °C in das Lösungsmittel eingebracht wird und/oder
iv) CO₂ in das Lösungsmittel der Lösung (A2) bis zu einer Konzentration von mindestens 3 g/l, vorzugsweise mindestens 4 g/l, noch mehr bevorzugt mindestens 4 g/l bis 8 g/l eingebracht wird und/oder
v) das Verfahren den weiteren Schritt c), Auflösen von mindestens einem Hilfsstoff (H2) in der Lösung (A2) umfasst und/oder
vi) bei Durchführung des Schrittes c), der mindestens eine Hilfsstoff (H2) ausgewählt ist aus einem Alkalihalogenid oder Erdalkalihalogenid, mehr bevorzugt aus NaCl und MgCl₂, noch mehr bevorzugt aus NaCl und/oder
vii) bei Durchführung des Schrittes c), 0 bis 20 mg/ml, vorzugsweise 3 bis 10 mg/ml des Hilfsstoffs (H2) bezogen auf das Gesamtvolumen der pharmazeutischen Zubereitung in Schritt c) in Lösung (A2) aufgelöst werden und/oder
viii) die Temperatur der Lösung (A2) in Schritt b) und/oder Schritt c) 0 bis 8 °C, mehr bevorzugt 0 bis 5 °C beträgt und/oder
ix) in Schritt b) 0,05 bis 100 mg/ml, vorzugsweise 0,08 bis 50 mg/ml, des Kohlensäure-Addukts (KA) in der Lösung (A2) gelöst werden.

13. Das Kohlensäure-Addukt (KA) gemäß einem der Ansprüche 1 bis 6 und 8 oder die pharmazeutische Zubereitung gemäß einem der Ansprüche 12i), 9 B), 9 A und 9 C) zur Verwendung in einem Verfahren zur Anästhesie, zur Analgesie, zur begleitenden Behandlung von Krebs, zur Entzündungshemmung, zur Unterstützung der Wundheilung, insbesondere bei Verbrennungen, offenen Wunden und Narben, zur Behandlung von neurogenen Entzündungen wie Multiple Sklerose, MMN (Multifokale motorische Neuropathie), zur Behandlung von Sinusitis, zur Behandlung von Asthma, zur Behandlung von Rheumatischer Arthritis, zur Behandlung von Alzheimer, zur Behandlung von Demenz, zur Unterstützung der Rekonvaleszenz und zur Unterstützung des Anti-aging, zur Behandlung des Burn-out-Syndroms, zur Behandlung von Arthrose, zur Behandlung von Polyarthritis, zur Behandlung von Schmerzsyndrom und allgemeinen Schmerzen, zur prä- und postoperativen Behandlung (auch bei Knochenbrüchen), zur Präventiv- und Rehabilitationsmedizin, zur Behandlung von Zosterneuralgie, zur begleitenden Behandlung von Zosterneuralgie, zur Behandlung von Erkrankungen der Bauchorgane, wie Leber, Galle, Bauchspeicheldrüse, Darm, zur Behandlung von Magen-Darm-Erkrankungen (Collitis Ulcerosa, Morbus Krohn), zur Behandlung von Morbus Bechterew, zur Behandlung von chron. Schmerzen des Bewegungsapparats, zur Behandlung von Diabetes (Verbesserung der Blutzuckerwerte), zur Reduktion von Ödemen, zur Comedikation zu Opioden oder anderen Analgetika.

14. Ein Kit zur Herstellung der pharmazeutischen Zubereitung gemäß Anspruch 9 C), umfassend
a) das Kohlensäure-Addukt (KA) gemäß einem der Ansprüche 1 bis 6 und 8,
b) die Lösung (A2), umfassend Wasser als Lösungsmittel und CO₂, vorzugsweise in einer Konzentration von mindestens 3 g/l, vorzugsweise mindestens 4 g/l, noch mehr bevorzugt mindestens 4 g/l bis 8 g/l und
c) den Hilfsstoff (H2), vorzugsweise ausgewählt aus einem Alkalihalogenid oder Erdalkalihalogenid, mehr bevorzugt aus NaCl und MgCl₂, noch mehr bevorzugt aus NaCl.

## Claims

1. Carbonic acid adduct (KA) comprising carbonic acid, at least one amine (AM) and optionally at least one salt (S),
which adduct can be prepared using a method comprising the steps of
a) providing a solution (A) which comprises at least one solvent and CO₂ dissolved in the at least one solvent,
optionally b) dissolving a base (BA) which does not correspond to the amine (AM) in the solution (A) so as to obtain the solution (A1),
c) dissolving the at least one amine (AM) in solution (A) or (A1) so as to obtain the solution (B),
d) freezing the solution obtained after the completion of step c),
e) storing the solution frozen in step d) at -100 to 0 °C for no longer than 4 days,
wherein the content of CO₂ in the solution subjected to step d) is at least 6 g/l, preferably at least 10 g/l, more preferably at least 12 g/l, even more preferably at least 14 g/l and very particularly preferably at least 15 g/l, and the amine (AM) can also be used in the form of a salt.

2. Carbonic acid adduct (KA) according to claim 1, wherein step a) comprises at least one of the following sub-steps:
a1) cooling the solvent, preferably water, to 3 to 8 °C, preferably 5 °C, and/or
a2) introducing CO₂ into the solvent, preferably up to a saturation concentration of 3 to 10 g/l, more preferably up to a saturation concentration of 4.5 to 7.5 g/l, the pH of the solution after saturation with CO₂ preferably being ≤ 3.0 to 6.0, even more preferably ≤ 4.3 to 4.8, and/or
a3) storing the solution (A) at 1 to 10 °C, preferably for at least 30 min, more preferably for at least 50 min, even more preferably for at least 60 min; up to a maximum of 5 d (120h); storage preferably taking place at 3 to 8 °C, preferably for at least 30 min, more preferably for at least 50 min, even more preferably for at least 60 min; up to a maximum of 5 d (120h);
step a) preferably comprising all sub-steps a1), a2) and a3), the sub-steps a1), a2) and a3) preferably being carried out in the order a2) following a1) and a3) following a2) and/or
wherein the base (BA) in step b) is a hydrogen carbonate or a carbonate, more preferably a hydrogen carbonate, even more preferably sodium hydrogen carbonate.

3. Carbonic acid adduct (KA) according to either of claims 1 to 2, wherein step c) comprises at least one of the following sub-steps:
c1) dissolving the at least one amine (AM) in solution (A) or (A1) so as to obtain the solution (B) and/or
c2) adding solution (A) to solution (B) so as to obtain the solution (B1) and/or
c3) enriching the solution (B) or (B1) with CO₂ and/or
c4) storing the solution (B) or (B1) at 1 to 10 °C, preferably 3 to 8 °C, for at least 1 h, preferably 24 h to 120 h, even more preferably 24 to 72 h, and/or
c5) enriching the solution (B) or (B1) with CO₂ to a concentration of at least 6 g/l, preferably at least 10 g/l, more preferably at least 12 g/l, even more preferably at least 14 g/l and very particularly preferably at least 15 g/l;
wherein optionally
i) the concentration of the amine (AM) in solution (B) or, if sub-step c2) is carried out, in solution (B1) is 0.01 to 0.25 g/ml, preferably 0.03 to 0.20 g/ml, more preferably 0.08 to 0.15 g/ml, and/or
ii) the pH of the solution (B) or (B1) after step c5) has been carried out is ≤ 7.0 and/or
iii) the ratio of the amine (AM) to the base (BA), when carrying out step b), in solution (B) is 2:1 to 5:1, more preferably 3:1 to 4:1, even more preferably 3.23:1 to 3.26:1 [g/g], and/or
iv) the molar ratio of the amine (AM) to the base equivalents of the base (BA), when carrying out step b), in solution (B) is 0.8:1 to 1.5:1, preferably 1.2:1, more preferably 1:1, and/or
v) in step c1), the at least one amine (AM) comprises the at least one amine (AM) as an acid addition salt, preferably as a hydrohalide, hydrogen sulfate, hydrogen sulfite, hydrogen phosphate, hydromesylate, hydrotosylate, hydroacetate, hydroformate, hydropropanoate, hydromalonate, hydrosuccinate, hydrofumarate, hydroxalate, hydrotartrate, hydrocitrate, hydromaleate, more preferably as hydrochloride or hydrobromide,
step c) preferably comprising all sub-steps c1), c2), c3), c4) and c5); the sub-steps c1), c2), c3), c4) and c5) preferably being carried out in the order c2) following c1), c3) following c2), c4) following c3), c5) following c4).

4. Carbonic acid adduct (KA) according to any of claims 1 to 3, wherein in step d):
i) the solution (B) or (B1) is frozen at -100 °C to -20 °C, preferably at -90 °C to -30 °C, even more preferably at -80 to -40 °C and very particularly preferably at -70 to - 50 °C, and/or
ii) the solution (B) or (B1) is frozen within 0.3 to 60 minutes, preferably within 1 to 30 minutes, more preferably within 1.1 to 10 minutes, even more preferably within 1.5 to 5 minutes, and/or
iii) the vessel in which the solution (B) or (B1) is located during the freezing process, preferably in the cooling medium, is rotated at 10 to 1000 rpm, preferably at 50 to 600 rpm, more preferably at 100 to 400 rpm and even more preferably at 200 to 300 rpm, and/or
iv) the solution (B) or (B1) is frozen at a cooling rate of 10 to 100 K/min, preferably at 20 to 80 K/min, more preferably at 30 to 70 K/min and particularly preferably at 40 to 60 K/min, and/or
wherein in step e):
i) the frozen solution (B) or (B1) is stored for 1.5 to 4 days, preferably for 2.5 to 4 days, and/or
ii) the frozen solution (B) or (B1) is preferably stored at -50 to 0 °C, more preferably at -30 to -5 °C, even more preferably at -25 to -10 °C, particularly preferably at -20 to -15 °C.

5. Carbonic acid adduct (KA) according to any of claims 1 to 4, wherein the method comprises a further step f) which is carried out after step e),
f) drying the solution stored in step e) so as to obtain dried carbonic acid adduct (KA), wherein in step f) optionally
i) the water from the solution (B) or (B1) is removed up to a residual content of < 0.8 wt.%, preferably < 0.1 wt.%, based on the total weight of the drying product (C), and/or
ii) CO₂ not bound in the carbonic acid adduct (KA) is removed from the solution (B) or (B1) up to a residual content of < 0.8 wt.%, preferably < 0.1 wt.%, based on the total weight of the drying product (C), and/or
iii) the drying is carried out by means of lyophilization and/or
iv) during drying, the pressure is 0.01 to 30 mbar, preferably 0.02 to 20 mbar, more preferably 0.03 to 10 mbar, even more preferably 0.03 to 0.5 mbar and very particularly preferably 0.05 to 0.1 mbar, and is preferably maintained during the entire drying process and/or
v) the pressure during drying according to iv) is reached within 10 h, preferably within 7 h, more preferably within 5 h and particularly preferably within 4 h from the start of evacuation, and/or
vi) the temperature during the entire drying process in step f) is 0 to 20 °C, preferably 4 to 18 °C, more preferably 8 to 16 °C, and/or
vii) the total drying time is 10 to 60 h, preferably 30 to 55 h, more preferably 41 to 52 h.

6. Carbonic acid adduct (KA) according to any of claims 1 to 5, wherein
a) at least one amine (AM) according to one of the following formulas (I) or (II) is used, 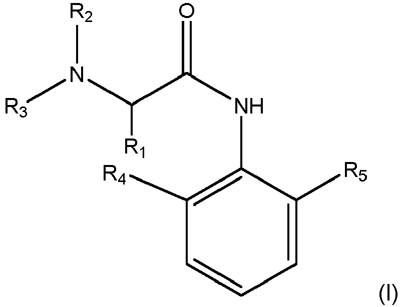 wherein in formula (I)
R₁ is H, C₁₋₁₀ alkyl, C₂₋₁₀ alkenyl, aryl or heteroaryl, preferably H or C₁₋₁₀ alkyl, more preferably H or C₁₋₄ alkyl;
R₂ is H, C₁₋₁₀ alkyl, C₂₋₁₀ alkenyl, aryl or heteroaryl, preferably H or C₁₋₁₀ alkyl, more preferably H or C₁₋₄ alkyl;
R₃ is H, C₁₋₁₀ alkyl, C₂₋₁₀ alkenyl, aryl or heteroaryl, preferably H or C₁₋₁₀ alkyl, more preferably H or C₁₋₄ alkyl;
wherein optionally R₁ and R₃ can be bound to one another and, together with the nitrogen atom to which R₃ is bound and the carbon atom to which R₁ is bound, can form a saturated or unsaturated ring, preferably a saturated ring, the ring preferably being 4, 5 or 6-membered, more preferably 5 or 6-membered,
R₄ is H, halogen, C₁₋₁₀ alkyl, C₂₋₁₀ alkenyl, aryl or heteroaryl, preferably H or methyl;
R₅ is H, halogen, C₁₋₁₀ alkyl, C₂₋₁₀ alkenyl, aryl or heteroaryl, preferably H, methyl or halogen; 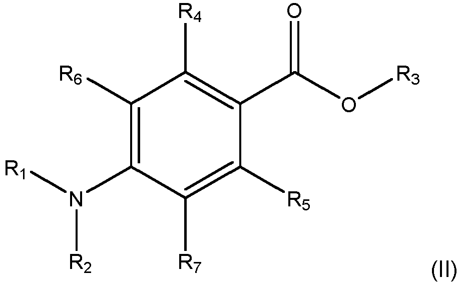 wherein in formula (II)
R₁ is H, C₁₋₁₀ alkyl, C₂₋₁₀ alkenyl, aryl or heteroaryl, preferably H or C₁₋₁₀ alkyl, more preferably H;
R₂ is H, C₁₋₁₀ alkyl, C₂₋₁₀ alkenyl, aryl or heteroaryl, preferably H or C₁₋₁₀ alkyl, more preferably H;
R₃ is H, C₁₋₁₀ alkyl, C₂₋₁₀ alkenyl, aryl, heteroaryl or -(CH₂)ₙNR₈R₉, preferably - (CH₂)ₙNR₈R₉;
n is 1 to 5, preferably 1 to 3, more preferably 1 to 2;
R₈ is C₁₋₁₀ alkyl, preferably C₁₋₂ alkyl;
R₉ is C₁₋₁₀ alkyl, preferably C₁₋₂ alkyl;
R₄ is H, halogen, C₁₋₁₀ alkyl, C₂₋₁₀ alkenyl, aryl, heteroaryl or -O-C₁₋₁₀ alkyl, preferably
H, halogen, C₁₋₁₀ alkyl or -O-C₁₋₁₀ alkyl, more preferably H or halogen;
R₅ is H, halogen, C₁₋₁₀ alkyl, C₂₋₁₀ alkenyl, aryl, heteroaryl or -O-C₁₋₁₀ alkyl, preferably
H, halogen, C₁₋₁₀ alkyl or -O-C₁₋₁₀ alkyl, more preferably H or halogen;
R₆ is H, halogen, C₁₋₁₀ alkyl, C₂₋₁₀ alkenyl, aryl, heteroaryl or -O-C₁₋₁₀ alkyl, preferably
H, halogen, C₁₋₁₀ alkyl or -O-C₁₋₁₀ alkyl, more preferably H or -O-C₁₋₁₀ alkyl;
R₇ is H, halogen, C₁₋₁₀ alkyl, C₂₋₁₀ alkenyl, aryl, heteroaryl or -O-C₁₋₁₀ alkyl, preferably
H, halogen, C₁₋₁₀ alkyl or -O-C₁₋₁₀ alkyl, more preferably H or -O-C₁₋₁₀ alkyl;
wherein the at least one amine according to formula (I) and (II) can optionally also be used in the form of a salt or
b) at least one amine (AM) is used, which amine is selected from the group of 4-aminobenzoic acid 2-(*N,N*-diethylamino)ethyl ester (procaine), 4-aminobenzoic acid ethyl ester (benzocaine), 2-(diethylamino)ethyl-4-amino-2-chlorobenzoate (chloroprocaine), 4-amino-3-butoxybenzoic acid 2-diethyl-aminoethyl ester (oxybuprocaine), (2-(dimethylamino)ethyl)-4-(butylamino)benzoate (tetracaine), *N-*[3-(4-phenoxymethylphenyl)propyl]morpholine (fomocaine), 2-diethylamino-*N*-(2,6-dimethylphenyl)acetamide (lidocaine), (*RS*)-*N*-(2,6-dimethylphenyl)-1-methylpiperidine-2-carboxamide (mepivacaine), (*RS*)-*N*-(2-methylphenyl)-2-(propylamino)propanamide (prilocaine), (*RS*)-4-methyl-3-[2-(propylamino)propanamido]thiophene-2-carboxylic acid methyl ester (articaine), (±)-1-butyl-*N*-(2,6-dimethylphenyl)-2-piperidinecarboxamide (bupivacaine), (*S*)-1-propyl-2',6'-dimethyl-2-piperidylcarboxyanilide (ropivacaine), 2-(ethylpropylamino)-2',6'-butyroxylidide (etidocaine), 1-(4-butoxyphenyl)-3-piperidin-1-ylpropan-1-one (dyclonine), preferably 4-aminobenzoic acid 2-(*N*,*N*-diethylamino)ethyl ester (procaine) and 2-diethylamino-*N*-(2,6-dimethylphenyl)acetamide (lidocaine), (2-(dimethylamino)ethyl)-4-(butylamino)benzoate (tetracaine) and/or the salts of these compounds.

7. Method for preparing the carbonic acid adduct (KA) according to any of claims 1 to 6, comprising the steps according to any of claims 1 to 6.

8. Carbonic acid adduct (KA) comprising at least one amine (AM), carbonic acid and optionally at least one salt (S), which adduct can be prepared using the method according to claim 7, wherein
i) the carbonic acid adduct (KA) comprises procaine, carbonic acid and at least one salt (S) and the decomposition point is 65 to 95 °C, preferably 70 to 90 °C, more preferably 85 to 90 °C, or comprises lidocaine, carbonic acid and at least one salt (S) and the decomposition point is 20 to 45 °C, preferably 25 to 45 °C, more preferably 30 to 45 °C, and/or
ii) the carbonic acid adduct (KA) remains stable when stored at a temperature of 2 to 10 °C for at least 12 months, preferably for at least 13 months, more preferably for at least 20 months, even more preferably for at least 23 months, particularly preferably for at least 27 months.

9. Pharmaceutical preparation (PZ) comprising the carbonic acid adduct (KA) according to any of claims 1 to 6 and 8, wherein optionally
i) when preparing the pharmaceutical preparation (PZ)
a) the temperature is less than 60 °C, preferably less than 50 °C, more preferably 0 °C to 50 °C, and/or
when using dispersers and/or ointment preparation agents, a rotational speed of < 2000 rpm is used;
wherein the pharmaceutical preparation (PZ) optionally
(A) is a semi-solid preparation and is administered cutaneously and the pharmaceutical preparation (PZ) optionally
i) is administered in the form of an ointment, a cream, a gel, a paste, a covering paste or a plaster containing active ingredient and/or
ii) comprises at least one auxiliary (H1) selected from paraffins, in particular high- and low-viscosity paraffins, wool wax, wool wax alcohols, hydrophobic base gel, vegetable oils, animal fats, synthetic glycerides, liquid polyalkylsiloxanes, waxes, petroleum jelly, starch, in particular corn starch, preferably petroleum jelly, and/or
iii) comprises
a) 0.1 to 40 wt.%, preferably 0.4 to 10 wt.%, of the carbonic acid adduct (KA) and
b) 60 to 99.9 wt.%, preferably 80 to 96 wt.%, of the auxiliary (H1), based on the total amount of the pharmaceutical preparation (PZ), or
(B) the pharmaceutical preparation (PZ) is administered orally and the pharmaceutical composition (PZ) optionally
i) is administered in capsules, preferably in hard gelatin or cellulose capsules, more preferably in hard gelatin capsules, and/or
ii) is administered in tablet form and/or
iii) comprises at least one auxiliary (H), preferably selected from starch, in particular corn starch and/or rice starch, dextran, cellulose ester and SiO₂, and/or
iv) comprises at least one base (BA1) which does not correspond to the amine (AM) and is identical or different from the base (BA), preferably the base (BA1) being selected from NaHCO₃ or KHCO₃, more preferably NaHCO₃, and/or;
v) comprises
a) 1 to 99 wt.%, preferably 15 to 95 wt.%, of the carbonic acid adduct (KA),
b) 0 to 60 wt.%, preferably 3 to 50 wt.%, of the base (BA1) and
c) 1 to 90 wt.%, preferably 2 to 75 wt.%, of the auxiliary (H), based on the total weight of the pharmaceutical preparation, or
(C) the pharmaceutical preparation (PZ) is administered parenterally, nasally and/or by inhalation and the pharmaceutical preparation (PZ) optionally
I) comprises an auxiliary (H2), preferably selected from an alkali halide or alkaline-earth halide, more preferably from NaCl and MgCl₂, even more preferably from NaCl, and/or
II) comprises
a) 0.05 to 100 mg/ml, preferably 0.08 to 50 mg/ml, of the carbonic acid adduct (KA), and
b) 0 to 20 mg/ml, preferably 3 to 10 mg/ml, of the auxiliary (H2), based in each case on the total volume of the pharmaceutical preparation (PZ).

10. Method for preparing the pharmaceutical preparation (PZ) according to claim 9 B), comprising the steps of:
a) providing a mixture comprising the carbonic acid adduct (KA) and optionally at least one base (BA1) and/or at least one auxiliary (H),
b) grinding the mixture to a powder,
c) processing the powder into a dosage form for oral administration, wherein optionally
i) the at least one auxiliary (H) is selected from starch, in particular corn starch and/or rice starch, dextran, cellulose ester and SiO₂, and/or
ii) the base (BA1) is selected from NaHCO₃ or KHCO₃, more preferably NaHCO₃, and/or
iii) the dosage form for oral administration is a tablet and/or
iv) the dosage form for oral administration is a capsule, preferably a hard gelatin or cellulose capsule, more preferably a hard gelatin capsule, and/or
v) 1 to 99 wt.%, preferably 15 to 95 wt.%, of the carbonic acid adduct (KA) is used, based on the total weight of the pharmaceutical preparation (PZ), and/or
vi) 0 to 60 wt.%, preferably 3 to 50 wt.%, of the base (BA1) is used, based on the total weight of the pharmaceutical preparation (PZ), and/or
vii) 1 to 90 wt.%, preferably 2 to 75 wt.%, of the auxiliary (H) is used, based on the total weight of the pharmaceutical preparation (PZ).

11. Method for preparing the pharmaceutical preparation (PZ) according to claim 9 A), comprising the steps of
a) grinding the carbon adduct (KA) to a powder,
b) mixing the carbon adduct (KA) powder from step a) with at least one auxiliary (H1), wherein optionally
i) the at least one auxiliary (H1) is selected from paraffins, in particular high- and low-viscosity paraffins, wool wax, wool wax alcohols, hydrophobic base gel, vegetable oils, animal fats, synthetic glycerides, liquid polyalkylsiloxanes, waxes, petroleum jelly, starch, in particular corn starch, preferably petroleum jelly, and/or
ii) the temperature in steps a) and b) is less than 60 °C, preferably less than 50 °C, more preferably 5 to 50 °C, and/or
iii) when using dispersers and/or ointment preparation agents, a rotational speed of < 2000 rpm is used and/or
iv) 0.1 to 40 wt.%, preferably 0.4 to 10 wt.%, of the carbonic acid adduct (KA) is used, based on the total amount of the pharmaceutical preparation, and/or
v) 60 to 99.9 wt.%, preferably 80 to 96 wt.%, of the auxiliary (H1) is used, based on the total amount of the pharmaceutical preparation (PZ).

12. Method for preparing the pharmaceutical preparation according to claim 9 C), comprising the steps of:
a) providing a solution (A2),
b) dissolving the carbonic acid adduct (KA) in the solution (A2), wherein optionally
i) the solution (A2) is preferably obtained by introducing CO₂ into a solvent and/or
ii) the solvent of the solution (A2) is water and/or
iii) CO₂ is introduced into the solvent at a temperature of 0 to 8 °C, more preferably at 0 to 5 °C, in order to prepare the solution (A2) and/or
iv) CO₂ is introduced into the solvent of the solution (A2) up to a concentration of at least 3 g/l, preferably at least 4 g/l, even more preferably at least 4 g/l to 8 g/l, and/or
v) the method comprises the further step c) of dissolving at least one auxiliary (H2) in the solution (A2) and/or
vi) when carrying out step c), the at least one auxiliary (H2) is selected from an alkali halide or alkaline-earth halide, more preferably from NaCl and MgCl₂, even more preferably from NaCl, and/or
vii) when carrying out step c), 0 to 20 mg/ml, preferably 3 to 10 mg/ml, of the auxiliary (H2) is dissolved in solution (A2) in step c), based on the total volume of the pharmaceutical preparation, and/or
viii) the temperature of the solution (A2) in step b) and/or step c) is 0 to 8 °C, more preferably 0 to 5 °C, and/or
ix) in step b) 0.05 to 100 mg/ml, preferably 0.08 to 50 mg/ml, of the carbonic acid adduct (KA) is dissolved in the solution (A2).

13. Carbonic acid adduct (KA) according to any of claims 1 to 6 and 8 or the pharmaceutical preparation according to any of claims 12i), 9 B), 9 A) and 9 C) for use in a method for anesthesia, for analgesia, for the concomitant treatment of cancer, for anti-inflammatory purposes, to support wound healing, in particular for burns, open wounds and scars, for the treatment of neurogenic inflammations such as multiple sclerosis and MMN (multifocal motor neuropathy), for the treatment of sinusitis, for the treatment of asthma, for the treatment of rheumatoid arthritis, for the treatment of Alzheimer's, for the treatment of dementia, to support convalescence and to support anti-aging, for the treatment of burn-out syndrome, for the treatment of osteoarthritis, for the treatment of polyarthritis, for the treatment of pain syndrome and general pains, for pre- and postoperative treatment (also for bone fractures), for preventive and rehabilitation medicine, for the treatment of zoster neuralgia, for the concomitant treatment of zoster neuralgia, for the treatment of diseases of the abdominal organs such as the liver, gall bladder, pancreas and intestine, for the treatment of gastrointestinal diseases (ulcerative colitis, Crohn's disease), for the treatment of ankylosing spondylitis, for the treatment of chronic musculoskeletal pain, for the treatment of diabetes (improving blood sugar levels), for reducing edema, and for comedication with opioids or other analgesics.

14. Kit for preparing the pharmaceutical preparation according to claim 9 C), comprising
a) the carbonic acid adduct (KA) according to any of claims 1 to 6 and 8,
b) the solution (A2), comprising water as the solvent and CO₂, preferably in a concentration of at least 3 g/l, preferably at least 4 g/l, even more preferably at least 4 g/l to 8 g/l, and
c) the auxiliary (H2), preferably selected from an alkali halide or alkaline-earth halide, more preferably from NaCl and MgCl₂, even more preferably from NaCl.

## Revendications

1. Produit d'addition d'acide carbonique (KA) comprenant de l'acide carbonique, au moins une amine (AM) et éventuellement au moins un sel (S),
pouvant être préparé selon un procédé comprenant les étapes
a) de fourniture d'une solution (A) qui comprend au moins un solvant et du CO₂ dissous dans l'au moins un solvant, éventuellement
b) de dissolution d'une base (BA), qui ne correspond pas à l'amine (AM), dans la solution (A) pour obtenir la solution (A1),
c) de dissolution de l'au moins une amine (AM) dans la solution (A) ou (A1) pour obtenir la solution (B),
d) de congélation de la solution obtenue à l'issue de l'étape c),
e) de conservation de la solution congelée à l'étape d) entre -100 et 0 °C pendant 4 jours maximum,
la teneur en CO₂ dans la solution soumise à l'étape d) étant d'au moins 6 g/l, de préférence d'au moins 10 g/l, plus préférablement d'au moins 12 g/l, de manière particulièrement préférée d'au moins 14 g/l et de manière tout particulièrement préférée d'au moins 15 g/l et l'amine (AM) pouvant également être utilisée sous forme de sel.

2. Produit d'addition d'acide carbonique (KA) selon la revendication 1, dans lequel l'étape a) comprend au moins l'une des sous-étapes suivantes :
a1) refroidissement du solvant, de préférence de l'eau, entre 3 et 8 °C, de préférence à 5 °C et/ou
a2) introduction de CO₂ dans le solvant, de préférence jusqu'à une concentration de saturation de 3 à 10 g/l, plus préférablement jusqu'à une concentration de saturation de 4,5 à 7,5 g/l, le pH de la solution après saturation en CO₂ étant de préférence ≤ 3,0 à 6,0, de manière particulièrement préférée ≤ 4,3 à 4,8 et/ou
a3) conservation de la solution (A) à 1 à 10 °C, de préférence pendant au moins 30 minutes, plus préférablement pendant au moins 50 minutes, de manière particulièrement préférée pendant au moins 60 minutes ; jusqu'à un maximum de 5 jours (120 h) ; la conservation s'effectuant de préférence entre 3 et 8 °C, de préférence pendant au moins 30 minutes, plus préférablement pendant au moins 50 minutes, de manière particulièrement préférée pendant au moins 60 minutes ; jusqu'à un maximum de 5 jours (120 h) ;
l'étape a) comprenant de préférence toutes les sous-étapes a1), a2) et a3), les sous-étapes a1), a2) et a3) étant de préférence réalisées dans l'ordre selon lequel a2) suit a1) et a3) suit a2) et/ou
la base (BA) à l'étape b) étant un hydrogénocarbonate ou un carbonate, plus préférablement un hydrogénocarbonate, de manière particulièrement préférée de l'hydrogénocarbonate de sodium.

3. Produit d'addition d'acide carbonique (KA) selon l'une des revendications 1 à 2, dans lequel l'étape c) comprend au moins l'une des sous-étapes suivantes :
c1) dissolution de l'au moins une amine (AM) dans la solution (A) ou (A1) pour obtenir la solution (B) et/ou
c2) ajout de la solution (A) à la solution (B) pour obtenir la solution (B1) et/ou
c3) enrichissement de la solution (B) ou (B1) en CO₂ et/ou
c4) conservation de la solution (B) ou (B1) entre 1 et 10 °C, de préférence entre 3 et 8 °C, pendant au moins 1 heure, de préférence pendant 24 heures à 120 heures, de manière particulièrement préférée pendant 24 heures à 72 heures, et/ou
c5) enrichissement de la solution (B) ou (B1) en CO₂ à une concentration d'au moins 6 g/l, de préférence d'au moins 10 g/l, plus préférablement d'au moins 12 g/l, de manière particulièrement préférée d'au moins 14 g/l et de manière tout particulièrement préférée d'au moins 15 g/l ;
dans lequel, éventuellement,
i) la concentration de l'amine (AM) dans la solution (B) ou, lors de la réalisation de la sous-étape c2), dans la solution (B1) est de 0,01 à 0,25 g/ml, de préférence de 0,03 à 0,20 g/ml, plus préférablement de 0,08 à 0,15 g/ml et/ou
ii) le pH de la solution (B) ou (B1) après la réalisation de l'étape c5) est ≤ 7,0 et/ou
iii) le rapport de l'amine (AM) à la base (BA), lors de la réalisation de l'étape b), dans la solution (B) est de 2:1 à 5:1, plus préférablement de 3:1 à 4:1, de manière particulièrement préférée de 3,23:1 à 3,26:1 [g/g] et/ou
iv) le rapport molaire de l'amine (AM) aux équivalents de base de la base (BA), lors de la réalisation de l'étape b), dans la solution (B) est de 0,8:1 à 1,5:1, de préférence de 1,2:1, plus préférablement de 1:1 et/ou
v) à l'étape c1), l'au moins une amine (AM) comprend l'au moins une amine (AM) en tant que sel d'addition d'acide, de préférence en tant qu'halohydrogénure, hydrogénosulfate, hydrogénosulfite, hydrogénophosphate, hydromésylate, hydrotosylate, hydroacétate, hydroformiate, hydropropanoate, hydromalonate, hydrosuccinate, hydrofumarate, hydroxalate, hydrotartrate, hydrocitrate, hydromaléate, plus préférablement en tant qu'hydrochlorure ou hydrobromure.
l'étape c) comprend de préférence toutes les sous-étapes c1), c2), c3), c4) et c5) ; les sous-étapes c1), c2), c3), c4) et c5) sont de préférence réalisées dans l'ordre selon lequel c2) suit c1), c3) suit c2), c4) suit c3), c5) suit c4).

4. Produit d'addition d'acide carbonique (KA) selon l'une des revendications 1 à 3, dans lequel, à l'étape d) :
i) la solution (B) ou (B1) est congelée entre -100 et -20 °C, de préférence entre -90 et -30 °C, de manière particulièrement préférée entre -80 et -40 °C et de manière tout particulièrement préférée entre -70 et -50 °C et/ou
ii) la solution (B) ou (B1) est congelée pendant 0,3 à 60 minutes, de préférence pendant 1 à 30 minutes, plus préférablement pendant 1,1 à 10 minutes, de manière particulièrement préférée pendant 1,5 à 5 minutes et/ou
iii) le récipient dans lequel se trouve la solution (B) ou (B1) est mis en rotation pendant le processus de congélation, de préférence dans un milieu de refroidissement, à 10 à 1 000 tr/min, de préférence à 50 à 600 tr/min, plus préférablement à 100 à 400 tr/min et de manière particulièrement préférée à 200 à 300 tr/min et/ou
iv) la solution (B) ou (B1) est congelée à une vitesse de refroidissement de 10 à 100 K/min, de préférence de 20 à 80 K/min, plus préférablement de 30 à 70 K/min et de manière particulièrement préférée de 40 à 60 K/min et/ou dans lequel, à l'étape e) :
i) la solution (B) ou (B1) congelée est conservée pendant 1,5 à 4 jours, de préférence pendant 2,5 à 4 jours et/ou
ii) la solution (B) ou (B1) congelée est conservée de préférence entre -50 et 0 °C, plus préférablement entre -30 et -5 °C, de manière particulièrement préférée entre -25 et -10 °C, de manière tout particulièrement préférée entre - 20 et -15 °C.

5. Produit d'addition d'acide carbonique (KA) selon l'une des revendications 1 à 4, dans lequel le procédé comprend une étape supplémentaire f), qui est réalisée après l'étape e),
f) séchage de la solution conservée à l'étape e) pour obtenir le produit d'addition d'acide carbonique (KA) séché,
dans lequel, éventuellement à l'étape f),
i) l'eau est éliminée de la solution (B) ou (B1) jusqu'à une teneur résiduelle < 0,8 % en poids, de préférence < 0,1 % en poids, par rapport au poids total du produit de séchage (C) et/ou
ii) le CO₂ non lié contenu dans le produit d'addition d'acide carbonique (KA) est éliminé de la solution (B) ou (B1) jusqu'à une teneur résiduelle < 0,8 % en poids, de préférence < 0,1 % en poids, par rapport au poids total du produit de séchage (C) et/ou
iii) le séchage est réalisé par lyophilisation et/ou
iv) pendant le séchage, la pression est maintenue entre 0,01 et 30 mbar, de préférence entre 0,02 et 20 mbar, plus préférablement entre 0,03 et 10 mbar, de manière particulièrement préférée entre 0,03 et 0,5 mbar et de manière tout particulièrement préférée entre 0,05 et 0,1 mbar et de préférence pendant tout le processus de séchage et/ou
v) la pression pendant le séchage selon iv) est atteinte dans les 10 heures, de préférence dans les 7 heures, plus préférablement dans les 5 heures et de manière particulièrement préférée dans les 4 heures à compter du début de l'évacuation et/ou
vi) la température pendant tout le séchage à l'étape f) est située entre 0 et 20 °C, de préférence entre 4 et 18 °C, plus préférablement entre 8 et 16 °C et/ou
vii) le temps de séchage total est de 10 à 60 heures, de préférence de 30 à 55 heures, plus préférablement de 41 à 52 heures.

6. Produit d'addition d'acide carbonique (KA) selon l'une des revendications 1 à 5, dans lequel
a) au moins une amine (AM) selon l'une des formules (I) ou (II) suivantes est utilisée, 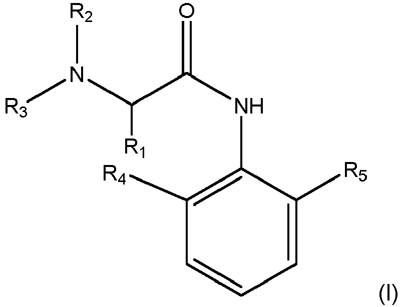 dans lequel, dans la formule (I),
R₁ représente H, un alkyle en C₁₋₁₀, un alcényle en C₂₋₁₀, un aryle ou un hétéroaryle, de préférence H ou un alkyle en C₁₋₁₀, plus préférablement H ou un alkyle en C₁₋₄ ;
R₂ représente H, un alkyle en C₁₋₁₀, un alcényle en C₂₋₁₀, un aryle ou un hétéroaryle, de préférence H ou un alkyle en C₁₋₁₀, plus préférablement H ou un alkyle en C₁₋₄ ;
R₃ représente H, un alkyle en C₁₋₁₀, un alcényle en C₂₋₁₀, un aryle ou un hétéroaryle, de préférence H ou un alkyle en C₁₋₁₀, plus préférablement H ou un alkyle en C₁₋₄ ;
R₁ et R₃ pouvant éventuellement être liés l'un à l'autre et pouvant éventuellement former un cycle saturé ou insaturé, de préférence un cycle saturé, de préférence un cycle à 4, 5 ou 6 chaînons, plus préférablement à 5 ou 6 chaînons, avec l'atome d'azote auquel R₃ est lié et l'atome de carbone auquel R₁ est lié,
R₄ représente H, un halogène, un alkyle en C₁₋₁₀, un alcényle en C₂₋₁₀, un aryle ou un hétéroaryle, de préférence H ou un méthyle ;
R₅ représente H, un halogène, un alkyle en C₁₋₁₀, un alcényle en C₂₋₁₀, un aryle ou un hétéroaryle, de préférence H, un méthyle ou un halogène ; 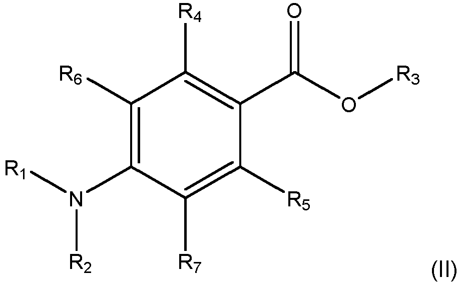 dans lequel, dans la formule (II),
R₁ représente H, un alkyle en C₁₋₁₀, un alcényle en C₂₋₁₀, un aryle ou un hétéroaryle, de préférence H ou un alkyle en C₁₋₁₀, plus préférablement H ;
R₂ représente H, un alkyle en C₁₋₁₀, un alcényle en C₂₋₁₀, un aryle ou un hétéroaryle, de préférence H ou un alkyle en C₁₋₁₀, plus préférablement H ;
R₃ représente H, un alkyle en C₁₋₁₀, un alcényle en C₂₋₁₀, un aryle, un hétéroaryle ou -(CH₂)ₙNR₈R₉, de préférence -(CH₂)ₙNR₈R₉,
n représente 1 à 5, de préférence 1 à 3, plus préférablement 1 à 2,
R₈ représente un alkyle en C₁₋₁₀, de préférence un alkyle en C₁₋₂,
R₉ représente un alkyle en C₁₋₁₀, de préférence un alkyle en C₁₋₂ ;
R₄ représente H, un halogène, un alkyle en C₁₋₁₀, un alcényle en C₂₋₁₀, un aryle, un hétéroaryle ou un alkyle en -O-C₁₋₁₀, de préférence H, un halogène, un alkyle en C₁₋₁₀ ou un alkyle en -O-C₁₋₁₀, plus préférablement H ou un halogène ;
R₅ représente H, un halogène, un alkyle en C₁₋₁₀, un alcényle en C₂₋₁₀, un aryle, un hétéroaryle, ou un alkyle en -O-C₁₋₁₀, de préférence H, un halogène, un alkyle en C₁₋₁₀ ou un alkyle en -O-C₁₋₁₀, plus préférablement H ou un halogène ;
R₆ représente H, un halogène, un alkyle en C₁₋₁₀, un alcényle en C₂₋₁₀, un aryle, un hétéroaryle, ou un alkyle en -O-C₁₋₁₀, de préférence H, un halogène, un alkyle en C₁₋₁₀ ou un alkyle en -O-C₁₋₁₀, plus préférablement H ou un alkyle en -O-C₁₋₁₀ ;
R₇ représente H, un halogène, un alkyle en C₁₋₁₀, un alcényle en C₂₋₁₀, un aryle, un hétéroaryle ou un alkyle en -O-C₁₋₁₀, de préférence H, un halogène, un alkyle en C₁₋₁₀ ou un alkyle en -O-C₁₋₁₀, plus préférablement H ou un alkyle en -O-C₁₋₁₀ ;
l'au moins une amine selon les formules (I) et (II) pouvant éventuellement être également utilisée sous la forme d'un sel ou
b) d'au moins une amine (AM), laquelle est choisie dans le groupe constitué par l'acide 4-aminobenzoate-2-(*N*,*N*-diéthylamino-)éthyle (procaïne), acide 4-aminobenzoate d'éthyle (benzocaïne), 2-(diéthyl-amino)éthyl-4-amino-2-chlorobenzoate (chloroprocaïne), acide 4-amino-3-butoxybenzoate-2-diéthyl-aminoéthyle (oxybuprocaïne), (2-(diméthylamino)éthyl)-4-(butylamino)benzoate (tétracaïne), N-[3-(4-phénoxyméthylphényl)propyl]morpholine (fomocaïne), 2-diéthylamino-*N*-(2,6-diméthylphényl)acétamide (lidocaïne), (*RS*)-*N*-(2,6-diméthylphényl)-1-méthylpipéridine-2-carboxamide (mépivacaïne), (*RS*)-*N*-(2-méthylphényl)-2-(propylamino)-propanamide (prilocaïne), (RS)-4-méthyl-3-[2-(propylamino)-propanamido]thiophène-2-carboxylate de méthyle (articaïne), (±)-1-butyl-*N*-(2,6-diméthylphényl)-2-pipéridinecarboxamide (bupivacaïne), (S)-1-propyl-2',6'-diméthyl-2-pipéridylcarboxyanilide (ropivacaïne), 2-(éthylpropylamino)-2',6'-butyroxylidide (étidocaïne), 1-(4-butoxyphényl)-3-pipéridin-1-ylpropan-1-one (dyclonine), de préférence 4-aminobenzoate-2-(*N*,*N*-diéthylamino-)éthyle (procaïne) et 2-diéthylamino-*N*-(2,6-diméthylphényl)acétamide (lidocaïne), (2-(diméthylamino)éthyl)-4-(butylamino)benzoate (tétracaïne) et/ou les sels de ces composés.

7. Procédé de préparation du produit d'addition d'acide carbonique (KA) selon l'une des revendications 1 à 6, comprenant les étapes selon l'une des revendications 1 à 6.

8. Produit d'addition d'acide carbonique (KA), comprenant au moins une amine (AM), de l'acide carbonique et éventuellement au moins un sel (S), pouvant être préparé selon le procédé selon la revendication 7,
i) le produit d'addition d'acide carbonique (KA),
comprenant de la procaïne, de l'acide carbonique et au moins un sel (S) et le point de décomposition étant de 65 à 95 °C, de préférence de 70 à 90 °C, plus préférablement de 85 à 90 °C ou
comprenant de la lidocaïne, de l'acide carbonique et au moins un sel (S) et le point de décomposition est de 20 à 45 °C, de préférence de 25 à 45 °C, plus préférablement de 30 à 45 °C et/ou
ii) le produit d'addition d'acide carbonique (KA) restant stable lorsqu'il est conservé à une température entre 2 et 10 °C pendant au moins 12 mois, de préférence pendant au moins 13 mois, plus préférablement pendant au moins 20 mois, de manière particulièrement préférée pendant au moins 23 mois, de manière tout particulièrement préférée pendant au moins 27 mois.

9. Préparation pharmaceutique (PZ) comprenant le produit d'addition d'acide carbonique (KA) selon l'une des revendications 1 à 6 et 8, dans laquelle, éventuellement,
i) lors de la préparation de la préparation pharmaceutique (PZ),
a) la température est inférieure à 60 °C, de préférence inférieure à 50 °C, plus préférablement entre 0 et 50 °C, et/ou
lors de l'utilisation de disperseurs et/ou d'onguents, une vitesse < 2000 tr/min est utilisée ;
la préparation pharmaceutique (PZ) étant éventuellement
(A) une préparation semi-solide et étant appliquée par voie cutanée, et la préparation pharmaceutique (PZ) éventuellement
i) étant appliquée sous forme d'une pommade, en tant que crème, gel, pâte, pâte enveloppante ou plâtre contenant un principe actif et/ou
ii) comprenant au moins une substance auxiliaire (H1) choisie parmi les paraffines, en particulier les paraffines épaisses et fines, la lanoline, les alcools de lanoline, le gel de base hydrophobe, les huiles végétales, les graisses animales, les glycérides synthétiques, les polyalkylsiloxanes liquides, les cires, la vaseline, l'amidon, en particulier l'amidon de maïs, de préférence la vaseline et/ou comprenant
iii)
a) 0,1 à 40 % en poids, de préférence 0,4 à 10 % en poids, du produit d'addition d'acide carbonique (KA) et
b) 60 à 99,9 % en poids, de préférence 80 à 96 % en poids, de la substance auxiliaire (H1) par rapport à la quantité totale de la préparation pharmaceutique (PZ) ou
(B) la préparation pharmaceutique (PZ) étant appliquée par voie orale, et la composition pharmaceutique (PZ) éventuellement
i) étant administrée en gélules, de préférence en gélules de gélatine dure ou de cellulose, plus préférablement en gélules de gélatine dure et/ou
ii) étant administrée sous forme de comprimés et/ou
iii) comprenant au moins une substance auxiliaire (H), de préférence choisie parmi l'amidon, en particulier l'amidon de maïs et/ou l'amidon de riz, le dextrane, l'ester de cellulose et le SiO₂ et/ou
iv) comprenant au moins une base (BA1) ne correspondant pas à l'amine (AM) et étant identique à ou différente de la base (BA), de préférence la base (BA1) étant choisie parmi NaHCO₃ ou KHCO₃, plus préférablement NaHCO₃ et/ou ;
v)
a) comprenant 1 à 99 % en poids, de préférence 15 à 95 % en poids, du produit d'addition d'acide carbonique (KA),
b) comprenant 0 à 60 % en poids, de préférence 3 à 50 % en poids, de la base (BA1) et
c) comprenant 1 à 90 % en poids, de préférence 2 à 75 % en poids, de la substance auxiliaire (H)
par rapport au poids total de la préparation pharmaceutique ou
(C) la préparation pharmaceutique (PZ) étant appliquée par voie parentérale, nasale et/ou par inhalation, et la préparation pharmaceutique (PZ) comprenant éventuellement
I) une substance auxiliaire (H2), de préférence choisie parmi un halogénure alcalin ou un halogénure alcalino-terreux, plus préférablement parmi NaCl et MgCl₂, de manière particulièrement préférée NaCl et/ou
II)
a) comprenant 0,05 à 100 mg/ml, de préférence 0,08 à 50 mg/ml, du produit d'addition d'acide carbonique (KA), et
b) comprenant 0 à 20 mg/ml, de préférence 3 à 10 mg/ml de la substance auxiliaire (H2) respectivement par rapport au volume total de la préparation pharmaceutique (PZ).

10. Procédé de préparation de la préparation pharmaceutique (PZ) selon la revendication 9 B), comprenant les étapes :
a) de fourniture d'un mélange comprenant le produit d'addition d'acide carbonique (KA) et éventuellement au moins une base (BA1) et/ou au moins une substance auxiliaire (H),
b) de broyage du mélange pour obtenir une poudre,
c) de transformation de la poudre en une forme galénique pour administration orale,
i) l'au moins une substance auxiliaire (H) étant éventuellement choisie parmi l'amidon, en particulier l'amidon de maïs et/ou l'amidon de riz, le dextrane, l'ester de cellulose et le SiO₂ et/ou
ii) la base (BA1) étant éventuellement choisie parmi NaHCO₃ ou KHCO₃, plus préférablement NaHCO₃ et/ou
iii) la forme galénique pour administration orale étant éventuellement un comprimé et/ou
iv) la forme galénique pour administration orale étant éventuellement une gélule, de préférence une gélule de gélatine dure ou de cellulose, plus préférablement une gélule de gélatine dure et/ou
v) le produit d'addition d'acide carbonique (KA) étant utilisé en une quantité de 1 à 99 % en poids, de préférence de 15 à 95 % en poids, par rapport au poids total de la préparation pharmaceutique (PZ), et/ou
vi) la base (BA1) étant utilisée en une quantité de 0 à 60 % en poids, de préférence de 3 à 50 % en poids, par rapport au poids total de la préparation pharmaceutique (PZ), et/ou
vii) la substance auxiliaire (H) étant utilisée en une quantité de 1 à 90 % en poids, de préférence de 2 à 75 % en poids, par rapport au poids total de la préparation pharmaceutique (PZ).

11. Procédé de préparation de la préparation pharmaceutique (PZ) selon la revendication 9 A), comprenant les étapes
a) de broyage du produit d'addition de carbone (KA) pour obtenir une poudre,
b) de mélange de la poudre de produit d'addition de carbone (KA) de l'étape a) avec au moins une substance auxiliaire (H1),
i) l'au moins une substance auxiliaire (H1) étant éventuellement choisie parmi les paraffines, en particulier les paraffines épaisses et fines, la lanoline, les alcools de lanoline, le gel de base hydrophobe, les huiles végétales, les graisses animales, les glycérides synthétiques, les polyalkylsiloxanes liquides, les cires, la vaseline, l'amidon, en particulier l'amidon de maïs, de préférence la vaseline et/ou
ii) la température à l'étape a) et b) étant éventuellement inférieure à 60 °C, de préférence inférieure à 50 °C, plus préférablement entre 5 et 50 °C et/ou
iii) lors de l'utilisation de disperseurs et/ou d'onguents, une vitesse < 2 000 tr/min étant éventuellement utilisée et/ou
iv) le produit d'addition d'acide carbonique (KA) étant éventuellement utilisé en une quantité de 0,1 à 40 % en poids, de préférence de 0,4 à 10 % en poids, par rapport à la quantité totale de la préparation pharmaceutique et/ou
v) la substance auxiliaire (H1) étant éventuellement utilisée en une quantité de 60 à 99,9 % en poids, de préférence de 80 à 96 % en poids, par rapport à la quantité totale de la préparation pharmaceutique (PZ).

12. Procédé de préparation de la préparation pharmaceutique selon la revendication 9 C), comprenant les étapes :
a) de fourniture d'une solution (A2),
b) de dissolution du produit d'addition d'acide carbonique (KA) dans la solution (A2),
i) la solution (A2) étant éventuellement de préférence obtenue en introduisant du CO₂ dans un solvant et/ou
ii) le solvant de la solution (A2) étant éventuellement de l'eau et/ou
iii) le CO₂ permettant la préparation de la solution (A2) étant éventuellement introduit dans le solvant à une température entre 0 et 8 °C, plus préférablement entre 0 et 5 °C, et/ou
iv) le CO₂ étant éventuellement introduit dans le solvant de la solution (A2) jusqu'à une concentration d'au moins 3 g/l, de préférence d'au moins 4 g/l, de manière particulièrement préférée d'au moins 4 g/l à 8 g/l et/ou
v) le procédé comprenant l'étape supplémentaire c) de la dissolution d'au moins une substance auxiliaire (H2) dans la solution (A2) et/ou
vi) lors de la réalisation de l'étape c), l'au moins une substance auxiliaire (H2) étant choisie parmi un halogénure alcalin ou un halogénure alcalino-terreux, plus préférablement parmi NaCl et MgCl₂, de manière particulièrement préférée parmi NaCl et/ou
vii) lors de la réalisation de l'étape c), 0 à 20 mg/ml, de préférence 3 à 10 mg/ml, de la substance auxiliaire (H2) par rapport au volume total de la préparation pharmaceutique à l'étape c) étant dissous dans la solution (A2) et/ou
viii) la température de la solution (A2) à l'étape b) et/ou à l'étape c) étant située entre 0 et 8 °C, plus préférablement entre 0 et 5 °C et/ou
ix) à l'étape b), 0,05 à 100 mg/ml, de préférence 0,08 à 50 mg/ml, du produit d'addition d'acide carbonique (KA) étant dissous dans la solution (A2).

13. Produit d'addition d'acide carbonique (KA) selon l'une des revendications 1 à 6 et 8 ou préparation pharmaceutique selon l'une des revendications 12i), 9 B), 9 A) et 9 C), destiné(e) à être utilisé(e) dans un procédé destiné à l'anesthésie, l'analgésie, le traitement concomitant du cancer, l'inhibition de l'inflammation, pour favoriser la cicatrisation des plaies, en particulier pour les brûlures, les plaies ouvertes et les cicatrices, pour le traitement des inflammations neurogènes telles que la sclérose en plaques, les NMM (neuropathies motrices multifocales), pour le traitement de la sinusite, pour le traitement de l'asthme, pour le traitement de la polyarthrite rhumatoïde, pour le traitement de la maladie d'Alzheimer, pour le traitement de la démence, pour favoriser la convalescence et pour favoriser l'antivieillissement, pour le traitement du syndrome du burn-out, pour le traitement de l'arthrose, pour le traitement de la polyarthrite, pour le traitement du syndrome douloureux et de la douleur générale, pour le traitement pré et postopératoire (également pour les fractures osseuses), pour la médecine préventive et de réadaptation, pour le traitement de la névralgie du zona, pour le traitement concomitant de la névralgie du zona, pour le traitement des maladies des organes abdominaux, tels que le foie, la vésicule biliaire, le pancréas, l'intestin, pour le traitement des maladies gastro-intestinales (rectocolite hémorragique, maladie de Crohn), pour le traitement de la spondylarthrite ankylosante, pour le traitement de la douleur chronique musculo-squelettique, pour le traitement du diabète (amélioration de la glycémie), pour la réduction des œdèmes, pour la comédication avec des opioïdes ou d'autres analgésiques.

14. Kit de préparation de la préparation pharmaceutique selon la revendication 9 C), comprenant
a) le produit d'addition d'acide carbonique (KA) selon l'une des revendications 1 à 6 et 8,
b) la solution (A2), laquelle comprend de l'eau comme solvant et du CO₂, de préférence à une concentration d'au moins 3 g/l, de préférence d'au moins 4 g/l, de manière particulièrement préférée d'au moins 4 g/l à 8 g/l et
c) la substance auxiliaire (H2), de préférence choisie parmi un halogénure alcalin ou un halogénure alcalino-terreux, plus préférablement parmi NaCl et MgCl₂, de manière particulièrement préférée NaCl.
